# EUROPEAN PATENT APPLICATION

(11) **EP 3 450 453 A1**
(43) Date of publication of application: **06.03.2019**
(21) Application number: 17886601.8
(22) Date of filing: 22.05.2017
(51) Int. Cl.: C07K 14/47, C12N 15/12, A61K 38/17, A61K 45/00, A61P 3/10, A61P 3/04, A61P 3/00

(54) **LEG1 PROTEIN, LEG1 GENE, APPLICATIONS OF LEG1 PROTEIN AND LEG1 GENE, AND DRUG**

(30) Priority: 27.12.2016 CN 201611229669; 27.12.2016 CN 201611229670; 27.12.2016 CN 201611227322; 27.12.2016 CN 201611227323
(71) Applicant: Zhejiang University, Hangzhou, Zhejiang 310058 (CN)
(72) Inventor: PENG, Jinrong, Hangzhou Zhejiang 310058 (CN); HU, Minjie, Hangzhou Zhejiang 310058 (CN)
(74) Representative: Adamson Jones
(86) International application number: PCT/CN2017/085350
(87) International publication number: WO 2018/120610

(57) **Abstract**

Provided are a Legl protein, a Legl gene, uses and drugs thereof, which relate to the technical field of the functions and uses of genes. Very comprehensive study has been conducted on the functions of mLeg1 gene by approaches in Genetics, Molecular Biology, Biochemistry and Cell biology, with mLeg1 knockout mice as the subject. The study results show that mLeg1 protein can regulate *in vivo* Akt signals through EGFR, and further regulate *in vivo* lipogenesis. The study results provide a new means and idea for the treatment of human obesity and physical recovery of cancer patients after chemotherapy by human intervention in the expression level of hLeg1 gene and hLeg1 protein in the later period.

Sequence List

<110>  Zhejiang University

<120>  Leg1 Protein, Leg1 Gene, and Uses and Drugs thereof

<130>  6090-4954-P-EP

<140>  PCT/CN2017/085350
<141>  2017-05-22

<150>  CN 201611229669
<151>  2016-12-27

<150>  CN 201611229670
<151>  2016-12-27

<150>  CN 201611227322
<151>  2016-12-27

<150>  CN 201611227323
<151>  2016-12-27

<160>  8

<170>  PatentIn version 3.5

<210>  1
<212>  PRT
<213>  Homo sapiens

<400>  1

Met Ala Phe Leu Pro Ser Trp Val Cys Val Leu Val Gly Ser Phe Ser
1               5                   10                  15
Ala Ser Leu Ala Gly Thr Ser Asn Leu Ser Glu Thr Glu Pro Pro Leu
            20                  25                  30
Trp Lys Glu Ser Pro Gly Gln Leu Ser Asp Tyr Arg Val Glu Asn Ser
        35                  40                  45
Met Tyr Ile Ile Asn Pro Trp Val Tyr Leu Glu Arg Met Gly Met Tyr
    50                  55                  60
Lys Ile Ile Leu Asn Gln Thr Ala Arg Tyr Phe Ala Lys Phe Ala Pro
65                  70                  75                  80
Asp Asn Glu Gln Asn Ile Leu Trp Gly Leu Pro Leu Gln Tyr Gly Trp
                85                  90                  95
Gln Tyr Arg Thr Gly Arg Leu Ala Asp Pro Thr Arg Arg Thr Asn Cys
            100                 105                 110
Gly Tyr Glu Ser Gly Asp His Met Cys Ile Ser Val Asp Ser Trp Trp
        115                 120                 125
Ala Asp Leu Asn Tyr Phe Leu Ser Ser Leu Pro Phe Leu Ala Ala Val
    130                 135                 140
Asp Ser Gly Val Met Gly Ile Ser Ser Asp Gln Val Arg Leu Leu Pro
145                 150                 155                 160
Pro Pro Lys Asn Glu Arg Lys Phe Cys Tyr Asp Val Ser Ser Cys Arg
                165                 170                 175
Ser Ser Phe Pro Glu Thr Met Asn Lys Trp Asn Thr Phe Tyr Gln Tyr
            180                 185                 190
Leu Gln Ser Pro Phe Ser Lys Phe Asp Asp Leu Leu Lys Tyr Leu Trp
        195                 200                 205
Ala Ala His Thr Ser Thr Leu Ala Asp Asn Ile Lys Ser Phe Glu Asp
    210                 215                 220
Arg Tyr Asp Tyr Tyr Ser Lys Ala Glu Ala His Phe Glu Arg Ser Trp
225                 230                 235                 240

Val Leu Ala Val Asp His Leu Ala Ala Val Leu Phe Pro Thr Thr Leu
                245                 250                 255
Ile Arg Ser Tyr Lys Phe Gln Lys Gly Met Pro Pro Arg Ile Leu Leu
            260                 265                 270
Asn Thr Asp Val Ala Pro Phe Ile Ser Asp Phe Thr Ala Phe Gln Asn
        275                 280                 285
Val Val Leu Val Leu Leu Asn Met Leu Asp Asn Val Asp Lys Ser Ile
    290                 295                 300
Gly Tyr Leu Cys Thr Glu Lys Ser Asn Val Tyr Arg Asp His Ser Glu
305                 310                 315                 320
Ser Ser Ser Arg Ser Tyr Gly Asn Asn Ser
                325                 330

<210>  2
<211>  337
<212>  PRT
<213>  Mus musculus

<400>  2

Met Ala Val Leu Ala Ser Trp Val Trp Val Leu Ala Gly Cys Phe Cys
1               5                   10                  15
Ala Ala Val Ala Glu Val Ser Asp Ser Ser Asp Pro Tyr Pro Pro Leu
            20                  25                  30
Trp Glu Asp Ser Pro Glu Gln Leu Ser Asp Tyr Met Met Glu Asp Gly
        35                  40                  45
Asn Tyr Ile Ile Asn Pro Trp Val Tyr Thr Asp Arg Met Gly Met Tyr
    50                  55                  60
Arg Ile Leu Leu Glu Glu Thr Ala Met Tyr Phe Ala Lys Tyr Gly Pro
65                  70                  75                  80
Glu Asn Glu Gln Asn Leu Leu Trp Gly Leu Pro Leu Gln Phe Gly Trp
                85                  90                  95
Gln Tyr Gln Ser Gly Arg Leu Ala Asp Pro Thr Gly Met Thr Asp Cys
            100                 105                 110
Gly Asn Glu Leu Asn Glu Ser Leu Cys Val Ser Val Asp Ser Trp Trp
        115                 120                 125
Ala Asp Ile Asn Tyr Tyr Leu Ser Val Ile Pro Phe Leu Ala Ala Val
    130                 135                 140
Asp Ser Gly Ile Thr Gly Ile Ser Pro Asn Gln Ile Thr Ile Leu Pro
145                 150                 155                 160
Pro Pro Lys Asp Gln Met Arg Phe Cys Tyr Asn Val Ser Asp Cys Gln
                165                 170                 175
Ser Ala Val Pro Gly Thr Met Asn Arg Trp Arg Asp Phe Phe Gln Tyr
            180                 185                 190
Met Gln Leu Asn Ser Ser Asp Phe Asp Gly Leu Leu Asn Tyr Leu Trp
        195                 200                 205
Glu Ala His Ala Ser Ser Leu Asp Tyr Pro Thr Ser Ala Phe Gly Asp
    210                 215                 220
Arg Tyr Asn Phe Tyr Ser Glu Lys Glu Ala Asn Phe Glu Glu Asn Trp
225                 230                 235                 240
Ala Ile Ala Val Asn Tyr Leu Ala Ala Ala Arg Leu Pro Thr Thr Gln
                245                 250                 255
Asn Arg Thr Tyr Ser Phe Gln Arg Gly Leu Pro Pro Arg Val Leu Val
            260                 265                 270
Asp Thr Asp Ile Ala Pro Phe Ile Pro Asp Phe Thr Pro Leu Gln Asn
        275                 280                 285
Glu Val Leu Val Ser Leu Lys Leu Leu Gly Asp Thr Asp Arg Asn Ser
    290                 295                 300
Gly Ser Leu Ser Leu Thr Leu Trp Glu Asn Leu Met Ser Thr Lys Leu
305                 310                 315                 320
Ala Arg Thr Leu Phe Leu Lys Ala Phe Glu Glu Phe Leu Ala Thr Ser
                325                 330                 335
Ser

<210> 3
<211> 49
<212> DNA
<213> Homo sapiens

<400> 3

atggcttttc ttccttcctg ggtttgtgta ctagttggtt ccttttctgc ttccttagca 60
gggacttcca atctctcaga gacagagccc cctctgtgga aggagagtcc tggtcagctc 120
agtgactaca gggtggagaa cagcatgtac attattaatc cctgggtata ccttgagaga 180
atggggatgt ataaaatcat attgaatcag acagccaggt attttgcaaa atttgcacca 240
gataatgaac agaatatttt atgggggttg cctctgcagt atggctggca atataggaca 300
ggcagattag ctgatccaac ccgaaggaca aactgtggct atgaatctgg agatcatatg 360
tgcatctctg tggacagttg gtgggctgat ttgaattatt ttctgtcttc attacccttt 420
cttgctgcgg ttgattctgg tgtaatgggg atatcatcag accaagtcag gcttttgccc 480
ccacccaaga atgagaggaa gttttgttat gatgtttcta gctgtcgttc atccttccct 540
gagacaatga acaagtggaa caccttttac cagtatttgc agtcaccttt tagtaagttt 600
gatgatctgt tgaagtactt atgggctgca cacacttcaa ccttggcaga taatatcaaa 660
agttttgaag acagatatga ttattattct aaagcagaag cgcatttgga gagaagttgg 720
gtactggctg tggatcattt agctgcagtc ctctttccta caaccttgat tagatcatat 780
aagttccaga agggcatgcc accacgaatt cttcttaata ctgatgtagc ccctttcatc 840
agtgacttta ctgcttttca gaatgtagtc ctggttcttc taaatatgct tgacaatgtg 900
gataaatcta taggttatct ttgtacagaa aaatctaatg tatatagaga tcattcggaa 960
tctagctcta gaagttatgg aaataactcc tga 993

<210> 4
<211> 1132
<212> DNA
<213> Mus musculus

<400> 4

atggctgtcc tggcttcctg ggtctgggta ctggctggct gcttctgtgc agctgtagca 60
gaggtttctg atagctcaga tccatatcct cctttatggg aagacagccc tgagcagttg 120
agtgactaca tgatggagga tgggaactac ataattaatc cttgggtata cactgacaga 180
atggggatgt acagaatctt actggaagag acagctatgt attttgcaaa atatggtcca 240
gaaaatgaac aaaatcttct ttggggattg cctctgcagt ttggctggca gtatcaatca 300
ggtagattag ctgatcccac tggaatgaca gactgtggca atgaacttaa tgagagtctg 360
tgtgtctctg tggacagttg gtgggcagat ataaattatt atctgagtgt gatacctttc 420
cttgctgctg ttgattctgg aataacagga atatcaccaa accaaatcac gatcctgcct 480
ccacccaagg atcagatgag gttttgttat aatgtttctg actgtcaatc tgccgtccct 540
ggaacaatga acagatggag agactttttt cagtacatgc agttgaattc cagtgacttt 600
gatggccttc ttaactactt atgggaagcc catgcctcat ccttggacta tcccaccagt 660
gcctttggag acagatataa tttctattct gaaaaagaag caaatttgga ggaaaactgg 720
gctattgctg tgaactattt agcggcagcc cgtcttccta caacccaaaa tagaacatac 780
agctttcaga ggggcctgcc cccacgagtc cttgttgata ctgacatagc cccttttatt 840
cctgacttca ctcctcttca gaacgaagtc ctggtatcac tcaaacttct tggtgatact 900
gacagaaatt caggatcatt atctttgact ctatgggaaa atctcatgag tacaaaactt 960
gcaagaacat tatttctaaa agcctttgaa gagttcctgg caacatcttc ttaa 1014

<210> 5
<211> 364
<212> PRT
<213> Danio rerio

<400> 5

Met Ser Glu Met Gly Phe Leu Arg Ser Val Ala Ala Val Leu Leu Leu
1               5                   10                  15
Ala Val Phe Ser His Ala Ala Val Val Thr Glu Asn Gly Leu Pro Ile
            20                  25                  30
Gln Trp Gly Lys Ala Pro Ser Asp Leu Ser His Leu Pro Ile Ser Asp

35                  40                  45
Thr Gly Val Gln Val Asn Pro Trp Asp Tyr Ser Gln Arg Met Thr Met
    50                  55                  60
Tyr Lys Met Leu Ile Asn Ala Thr Asn Ala Tyr Met Ser Ser Met Gly
65                  70                  75                  80
Pro Gly Glu Gln Glu Asn Pro Leu Trp Ser Leu Pro Leu Gln Leu Gly
                85                  90                  95
Trp Lys Leu Lys Ser Gly Arg Leu Ala Asp Pro Thr Leu Asp Ser Ser
            100                 105                 110
Ser Thr Cys Gly Ser Glu Ala Ser Asp Pro Val Cys Ile Ser Pro Leu
        115                 120                 125
Ser Trp Phe Ala Cys Val Asn Tyr Tyr Leu Ser Val Leu Pro Phe Leu
    130                 135                 140
Ala Ala Val Glu Thr Gly Val Val Ser Ser Gly Gly His Gln Val Leu
145                 150                 155                 160
Ile Gln Val Pro Ala Glu Val Ala Gln Asp Tyr Cys Ser Ser Tyr Ser
                165                 170                 175
Asp Cys Ser Thr Lys His Pro Asn Ala Met Ala Lys Trp His Leu Phe
            180                 185                 190
Phe Gln Ser Leu Arg Gln Val Ser Gln Ser Glu Asp Ser Asp Phe Asn
        195                 200                 205
Lys Lys Asp Ser Ile Leu Gly Leu Met Trp Ala Ala Glu Glu Glu Ser
    210                 215                 220
Leu Gln Thr Ala Ser Gly Ala Cys Thr Glu Arg Gln Lys Leu Tyr Ser
225                 230                 235                 240
Ser Pro Glu Val Ser Phe Gln Gln Ser Trp Leu Asn Ser Ala Ala Phe
                245                 250                 255
Val Ser Ala Ala His Phe His Ala Asn Ile Glu Arg Ser Glu Lys Phe
            260                 265                 270
Met Ala Pro Leu Pro Ser Arg Val Leu Gln Glu Ala Asp Ser Pro Pro
        275                 280                 285
Asn Ile Ala Asp Leu Ser Thr Glu Glu Asn His Thr Leu Tyr Ile Phe
    290                 295                 300
Gly Trp Met Asn Ser Val Asn Gln Leu Leu Gly Gly Ser Leu Val Asn
305                 310                 315                 320
Leu Trp Arg Lys Ala Met Cys Ser Ala Gln Ala Arg Glu Lys Gly Gln
                325                 330                 335
Ala Leu Leu His Asp Leu Ile Leu Asp Pro Lys Phe Pro Gly Ser Ser
            340                 345                 350
Leu Trp Ser Ile Leu Ser Glu Met Ser Thr Ser Cys
        355                 360

<210>  6
<211>  364
<212>  PRT
<213>  Danio rerio

<400>  6

Met Ser Glu Met Gly Phe Leu Arg Ser Val Ala Ala Val Leu Leu Leu
1               5                   10                  15
Ala Val Phe Ser His Ala Ala Val Val Thr Glu Asn Gly Leu Pro Ile
            20                  25                  30
Gln Trp Arg Lys Ala Pro Ser Asp Leu Ser His Leu Pro Ile Ser Asn
        35                  40                  45
Thr Gly Val Gln Val Asn Pro Trp Val Tyr Ser Gln Arg Met Thr Met
    50                  55                  60
Leu Lys Met Val Ile Asn Ala Thr Asn Ala Tyr Met Ser Ser Met Gly
65                  70                  75                  80
Pro Gly Glu Gln Glu Asn Pro Leu Trp Ser Leu Pro Leu Gln Leu Gly
                85                  90                  95
Trp Lys Leu Lys Ser Gly Arg Leu Ala Asp Pro Thr Pro Asp Ser Ser
            100                 105                 110

Ser Thr Cys Gly Ser Glu Ala Ser Asp Pro Val Cys Ile Ser Pro Leu
        115                 120                 125
Ser Phe Phe Gly Cys Val Asn Tyr Tyr Leu Ser Val Leu Pro Phe Leu
    130                 135                 140
Ala Ala Val Glu Thr Gly Val Val Ser Ser Gly Gly His Gln Met Leu
145                 150                 155                 160
Ile Gln Val Pro Ala Glu Val Ala Gln Asp Tyr Cys Ser Ser Tyr Ser
                165                 170                 175
Asp Cys Ser Thr Lys His Pro Asn Thr Met Ala Lys Trp His Leu Phe
            180                 185                 190
Phe Gln Ser Leu Arg Gln Val Ser Gln Ser Lys Glu Ser Asp Phe Asn
        195                 200                 205
Lys Lys Asp Ser Ile Leu Gly Leu Met Trp Ala Ala Glu Glu Glu Ser
    210                 215                 220
Ile Gln Thr Ala Ser Gly Ala Cys Thr Glu Arg Gln Lys Leu Tyr Ser
225                 230                 235                 240
Ser Pro Glu Val Ser Phe Gln Gln Ser Ser Val Asn Leu Gly Ala Phe
                245                 250                 255
Ile Ser Ala Ala His Tyr His Ala Ser Ile Glu Arg Ser Gly Lys Phe
            260                 265                 270
Met Ser His Leu Pro Ser Arg Val Leu Gln Glu Ala Asp Ser Pro Pro
        275                 280                 285
Asn Ile Ala Asp Leu Ser Thr Glu Glu Asn His Ala Leu Tyr Met Leu
    290                 295                 300
Ser Trp Met Asn Ser Ile Asn Gln Leu Leu Gly Gly Ser Leu Val Asp
305                 310                 315                 320
Leu Trp Arg Lys Ala Met Cys Ser Ala Gln Ala Arg Glu Lys Gly Gln
                325                 330                 335
Ala Phe Leu Asp Asp Leu Ile Leu Asp Pro Lys Phe Pro Gly Ser Ser
            340                 345                 350
Leu Trp Ser Ile Ile Cys Val Met Ser Thr Ser Cys
        355                 360

<210>  7
<211>  355
<212>  PRT
<213>  Ovis aries

<400>  7

Met Trp Ser Ser Leu Val Ile Pro Thr Phe Leu Phe Leu Ser Phe Ser
1               5                   10                  15
Ser Val Gly Leu Ala Pro Asp Pro Gly Ser Thr Thr His Glu Asn Asp
            20                  25                  30
Asn Tyr Pro Pro Phe Trp Gly Gln Thr Asp Gly Asp Ile Ala Glu Phe
        35                  40                  45
Pro Val Gln Asn Asn Lys Ile Ile Val Asp Pro Trp Lys Tyr Met Asp
    50                  55                  60
Arg Leu Arg Ile Phe Lys Ile Leu Ile Thr Glu Ser Asn Lys Tyr Phe
65                  70                  75                  80
Ala Ser Phe Gly Lys Asn Asp Thr Gly Asn Val Phe Trp Ala Leu Thr
                85                  90                  95
Leu Leu Tyr Gly Ser Leu Phe Lys Ser Asp Arg Phe Ser Glu Pro Pro
            100                 105                 110
Asn Ser Ser Arg Cys Ala Tyr Glu Ser Gly Val Ser Ser Cys Ile Ser
        115                 120                 125
Ile Asn Ser Gly Trp Gly Gly Ile Ser Tyr Tyr Val Val Ile Met Tyr
    130                 135                 140
Phe Leu Ala Ala Ile Glu Ser Glu Phe Leu Gly Asn Leu Pro Tyr Glu
145                 150                 155                 160
Val Glu Leu Leu Ser Arg Lys Glu Tyr Arg Ser Asn Phe Cys Tyr Ser
                165                 170                 175
Val Glu Glu Cys Arg Ala Ala Tyr Pro Gln Ala Met Asp Ile His Asn

180                 185                 190
Arg Phe Tyr Lys Tyr Leu Gln Ser Arg Lys Ile Val Ser Thr Thr Ser
        195                 200                 205
Gly Ile Pro Gln Tyr Asn Thr Asp Glu Asp Thr Ala Ile Phe Lys Met
    210                 215                 220
Trp Ala Ala His Gln Ala Ala Leu Asp Val Ala Lys Pro Lys Phe Arg
225                 230                 235                 240
Asp Val Ser Phe Tyr Ser Ser Glu Thr Glu Arg Asp Phe Thr Met Asp
                245                 250                 255
Phe Leu Leu Ala Ala Glu Phe Ile Glu Ala Val Leu Tyr Arg Pro Tyr
            260                 265                 270
Phe Glu Ser Ser Ala Glu Phe Leu Ala Gly Phe Pro His Arg Leu Leu
        275                 280                 285
Thr Asp Gln Asp Arg Asn Val Leu Thr Ser Asn Phe Ser Arg Arg Glu
    290                 295                 300
Lys Ala Leu Ile Thr Val Val Lys Leu Ile Thr Lys Ile Asn Lys Ser
305                 310                 315                 320
Thr Gly Gly Leu Leu Leu Thr Ile Trp Lys Lys Leu Met Thr Ser Lys
                325                 330                 335
Phe Ala Arg Ala Met Gly Arg Phe Phe Ile Lys Arg Leu Leu Leu Ile
            340                 345                 350
Pro Val Glu
        355

<210>  8
<211>  317
<212>  PRT
<213>  Bos taurus

<400>  8

Pro Pro Phe Trp Asp Gln Ile Asp Gly Asp Ile Ala Glu Phe Pro Val
1               5                   10                  15
Gln Asn Asn Lys Ile Ile Val Asp Pro Trp Lys Tyr Met Asp Arg Leu
            20                  25                  30
Arg Ile Phe Lys Ile Leu Ile Thr Glu Ser Asn Lys Tyr Phe Ala Ser
        35                  40                  45
Phe Gly Lys Asn Asn Thr Gly Asn Val Phe Trp Ala Leu Thr Leu Leu
    50                  55                  60
Tyr Gly Arg Leu Phe Lys Thr Asp Arg Phe Ala Glu Pro Pro Asn Ser
65                  70                  75                  80
Ser Arg Cys Ala Tyr Glu Ser Gly Ile Ser Ser Cys Ile Ser Ile Asn
                85                  90                  95
Ser Gly Trp Gly Gly Ile Ser Tyr Tyr Val Val Met Met Tyr Phe Leu
            100                 105                 110
Ala Ala Ile Glu Ser Glu Phe Leu Gly Ser Leu Pro Tyr Glu Val Glu
        115                 120                 125
Leu Leu Ser Arg Glu Glu Tyr Arg Ser Asn Phe Cys Tyr Ser Ile Glu
    130                 135                 140
Glu Cys Arg Ala Ala His Pro Gln Ile Met Asp Ile Ala Asn Arg Phe
145                 150                 155                 160
Tyr Lys Gln Thr Lys Lys Ile Val Ser Thr Thr Ser Gly Ile Pro Gln
                165                 170                 175
Tyr Asp Thr Asp Glu Asp Thr Ala Ile Phe Lys Met Trp Ala Ala His
            180                 185                 190
Gln Ala Ala Ile Asp Val Ala Lys Pro Met Phe Ser Asp Val Ser Phe
        195                 200                 205
Tyr Ser Ser Glu Thr Glu Arg Asp Phe Thr Met Asp Phe Leu Leu Ala
    210                 215                 220
Ala Glu Phe Phe Glu Ala Ala Leu Tyr Arg Pro Tyr Phe Glu Ser Ser
225                 230                 235                 240
Ala Glu Phe Leu Val Gly Phe Pro His Arg Leu Leu Thr Asp Gln Asp
                245                 250                 255

Arg Asn Val Leu Met Ser Asn Phe Ser Arg Arg Glu Lys Ala Leu Ile
            260                 265                 270
Thr Val Ala Lys Leu Thr Thr Lys Ile Asn Lys Ser Thr Gly Gly Leu
        275                 280                 285
Leu Leu Thr Ile Trp Lys Lys Leu Met Thr Ser Lys Phe Ala Arg Ala
    290                 295                 300
Thr Gly Arg Phe Phe Ile Lys Arg Leu Leu Leu Ile Pro
305                 310                 315

## Description

The present application claims the priority of the Chinese patent application No. CN201611227322.5, entitled "Leg1 Protein and Use thereof in Obesity-related diseases", the priority of the Chinese patent application No. CN201611227323X, entitled "Leg1 Protein, Leg1 Gene and Uses thereof in Non-human", the priority of the Chinese patent application No. CN201611229669.3, entitled "hLeg1 Protein, Use and Drug thereof", and the priority of the Chinese patent application No. CN201611229670.6, entitled "hLeg1 Gene, Use and Drug thereof", which applications were filed with the Chinese Patent Office on December 27, 2016, and the entire contents of which are incorporated herein by reference.

### Technical Field

The present disclosure relates to the technical field of the functions and uses of genes, and particularly to a Legl protein, a Legl gene, and uses and drugs thereof.

### Background Art

In the past several years, obesity cases have increased rapidly around the world, and obesity currently has become No. 5 health threat causing death of humans. In developed countries, obesity was initially exposed in the 1980s, then the cases thereof continuously increased, and the increase has just slowed down in the past eight years; while in developing countries, the obesity patients increase exceedingly rapidly every year. Although obesity generally does not directly lead to death, complications caused by obesity, especially cardiovascular diseases and diabetes, can be fatal. In 2010, about 3.4 million people died as a result of obesity. In addition, the research on the patients suffering from obesity in the United States shows that obesity is highly likely to reduce average lifespan of people in the future. According to statistics, in order to treat obesity, the United States spend almost 117 billion USD every year. Furthermore, more and more attention has being paid to obesity around the world. However, at present, there are still relatively few clinically available drugs for effectively treating obesity, and also relatively few known drug targets related to obesity.

In view of the above, the present disclosure is proposed.

### Disclosure of the Invention

An object of the present disclosure is to provide a Legl protein, a Legl gene, uses and drugs thereof. The study results of the present disclosure show that the Legl protein and the Legl gene are closely related to *in vivo* lipogenesis, which provide a brand-new drug target and a new therapeutic approach and idea for the development of drugs in the fields of treatment or prevention of obesity, and for physical recovery of cancer patients after chemotherapy, treatment of lipopenia, weight gaining, treatment of diabetes, detection of salivary gland diseases, etc.

In the first aspect, the present disclosure provides a Legl protein having an amino acid sequence as set forth in (1), (2), (3) or (4):
(1) SEQ ID NO: 1;
(2) SEQ ID NO: 2;
(3) a derivative sequence that is obtained by subjecting the sequence as set forth in SEQ ID NO: 2 to substitution and/or deletion and/or addition of a plurality of amino acid residues and has the same or antagonizing bioactivity as SEQ ID NO: 2;
(4) a derivative sequence that is obtained by subjecting the sequence as set forth in SEQ ID NO: 2 to substitution, deletion or addition of one amino acid residue and has the same or antagonizing bioactivity as SEQ ID NO: 2.

When the amino acid sequence of the Legl protein is as set forth in SEQ ID NO: 1, the Legl protein is a human hLeg1 protein;
when the amino acid sequence of the Legl protein is as set forth in SEQ ID NO: 2, the Legl protein is a mouse mLeg1 protein;
when the amino acid sequence of the Legl protein is as set forth in a derivative sequence that is obtained by subjecting the sequence as set forth in SEQ ID NO: 2 to substitution and/or deletion and/or addition of a plurality of amino acid residues or by subjecting the sequence as set forth in SEQ ID NO: 2 to substitution, deletion or addition of one amino acid residue, and has the same or antagonizing bioactivity as SEQ ID NO: 2, the Legl protein can be a zebrafish dLegla protein (the amino acid sequence thereof is as set forth in SEQ ID NO: 5) or a zebrafish dLeglb protein (the amino acid sequence thereof is as set forth in SEQ ID NO: 6), a sheep oLeg1 protein (the amino acid sequence thereof is as set forth in SEQ ID NO: 7), a bovine bLeg1 protein (the amino acid sequence thereof is as set forth in SEQ ID NO: 8), or a protein of other vertebrates having homology with the Legl protein.

In conjunction with the first aspect, the present disclosure further provides a use of the Legl protein.

The present disclosure provides a use of the Legl protein as a target protein in the preparation or screening of a drug for treating obesity or reducing weight, the drug being a drug inhibiting the level of the Legl protein; or the drug being a drug blocking the binding of the Legl protein to the epidermal growth factor receptor (EGFR) protein; or the drug being a drug inhibiting the activity of the Legl protein.

The present disclosure further provides a use of the Legl protein as a target protein in the preparation or screening of a drug for treating lipopenia or gaining weight, the drug being a drug enhancing the level of the Legl protein; or the drug being a drug promoting the binding of the Legl protein to the EGFR protein; or the drug being a drug enhancing the activity of the Legl protein.

The present disclosure further provides a use of the Legl antibody used to inhibit Legl function for treating obesity.

The present disclosure further provides a use of the Legl protein for treating lipopenia or gaining weight.

The present disclosure further provides a use of the Legl protein as a target protein in the preparation or screening of a drug for treating human diabetes, the drug being a drug activating an Akt signaling pathway by enhancing the level of the Legl protein or enhancing the activity of the Legl protein to make GLUT2 transported to a surface of a cell membrane.

A further object of the present disclosure is to provide a use of the Legl protein as a marker in the preparation of a kit for detecting a morphological structure or diseases of salivary glands. It is shown by the study of the present disclosure that Legl protein is specifically expressed mainly in salivary glands, and is then delivered to liver to take action. Therefore, with the Legl protein as a marker, the expression level of the Legl protein can serve as a basis for diagnosing the morphological structure or diseases of salivary glands, so as to determine whether the morphological structure of the salivary glands is normal or whether a disease related to the salivary glands is developed.

In the second aspect, the present disclosure further provides a Legl gene encoding the Legl protein in the first aspect.

According to degeneracy of codons, in the case where the amino acid sequence of the Legl protein is known, the nucleotide sequence of the Legl gene encoding the Legl protein is not unique. Therefore, all the nucleotide sequences capable of encoding the Legl protein and the uses of these nucleotide sequences fall within the protection scope of the present disclosure.

Preferably, when the amino acid sequence of the Legl protein is as set forth in SEQ ID NO: 1, the nucleotide sequence of the Legl gene is as set forth in SEQ ID NO: 3; and when the amino acid sequence of the Legl protein is as set forth in SEQ ID NO: 2, the nucleotide sequence of the Legl gene is as set forth in SEQ ID NO: 4.

In conjunction with the second aspect, the present disclosure further provides a use of the Legl gene.

The present disclosure provides a use of the Legl gene as a target gene in the preparation or screening of a drug for treating obesity or reducing weight, the drug being a drug inhibiting the expression level of the Legl gene.

The present disclosure further provides a use of the Legl gene as a target gene in the preparation or screening of a drug for treating lipopenia or gaining weight, the drug being a drug enhancing the expression level of the Legl gene.

The present disclosure further provides a use of the Legl gene as a target gene in the preparation or screening of a drug for treating human diabetes, the drug being a drug activating an Akt signaling pathway making by enhancing the expression level of the Legl gene, so as to stimulate the transportation of GLUT2 to a surface of a cell membrane.

The present disclosure further provides a use of an RNA interference vector of the Legl gene in the preparation of a drug for treating obesity or reducing weight, the RNA interference vector silencing the expression of the Legl gene.

The present disclosure further provides a use of the Legl gene as a target gene in the preparation of a drug for regulating fat accumulation in vertebrates, the drug being a drug enhancing the expression level of the Legl gene; or the drug being a drug reducing or silencing the expression level of the Legl gene. It should be noted that when the drug is a drug enhancing the expression level of the Legl gene, the drug can promote fat accumulation in vertebrates; and when the drug is a drug reducing or silencing the expression level of the Legl gene, the drug can inhibit fat accumulation in vertebrates.

The present disclosure further provides a use of the Legl gene in breeding a high-fat-content vertebrate strain, comprising: introducing a plasmid vector containing the Legl gene into a target animal via gene engineering approach, the Legl gene being driven to be expressed by a strong promoter. It can be easily understood that, by means of the genetic engineering technology, the level of the Legl protein is improved through the overexpression effect of the Legl gene in the corresponding vertebrate, which thereby can improve fat accumulation in the vertebrate, and can further breed vertebrate strains with a high fat mass.

The present disclosure further provides use of the Legl gene in breeding a low-fat-content vertebrate strain, comprising: knocking out or knock down the Legl gene from the vertebrate strain. It can be easily understood that, by means of the genetic engineering and gene editing technology, the Legl gene in the vertebrate is knocked out to reduce the level of the Legl protein, which thereby can inhibit fat accumulation in the vertebrate, and breed invertebrate strains with a low fat content.

In the third aspect, the present disclosure further provides a recombinant Legl protein, obtained by subjecting the Legl gene provided in the second aspect to recombinant expression and purification in a prokaryotic expression system.

Further, the prokaryotic expression system is any of an *Escherichia coli* expression system, a *Bacillus subtilis* expression system and a *Streptomyce* expression system.

In conjunction with the third aspect, the present disclosure provides use of the recombinant Legl protein for treating obesity or reducing weight.

In the fourth aspect, the present disclosure further provides a modified Legl protein, obtained by modifying one or more amino acid residues in the Legl protein provided in the first aspect, the modification being one or more of glycosylation modification, acetylation modification, methylation modification and phosphorylation modification.

In conjunction with the fourth aspect, the present disclosure provides use of the modified Legl protein.

The modified Legl protein is used for treating obesity or reducing weight, treating human diabetes, treating lipopenia, or promoting fat accumulation in human body.

In the fifth aspect, there is provided a drug for treating obesity or reducing weight, the drug being a drug inhibiting the level of the Legl protein provided in the first aspect, with the Legl protein as the target, wherein inhibiting the level of the Legl protein can be construed as: inhibiting the expression level of the Legl protein; or inhibiting the basal level of the Legl protein, i.e., degrading the Legl protein so as to reduce the content of the Legl protein.

Or the drug is a drug blocking binding of the Legl protein to the EGFR protein, with the Legl protein as the target;
or the drug is a drug inhibiting the activity of the Legl protein, with the Legl protein as the target;
or the drug is a drug inhibiting the expression level of the Legl gene provided in the second aspect, with the Legl gene as the target;
or the drug is an RNA interference vector for silencing the Legl gene expression.

In the sixth aspect, the present disclosure provides a drug for treating lipopenia or gaining weight, the active ingredient of the drug being the Legl protein provided in the first aspect;
or the drug being a drug enhancing the level of the Legl protein, with the Legl protein as the target;
or the drug being a drug promoting the binding of the Legl protein to the EGFR protein, with the Legl protein as the target;
or the drug being a drug enhancing the activity of the Legl protein, with the Legl protein as the target;
or the drug being a drug enhancing the expression level of the Legl gene provided in the second aspect, with the Legl gene as the target.

In the seventh aspect, the present disclosure provides a drug for treating human diabetes, the active ingredient thereof being the Legl protein provided in the first aspect;
or the drug is a drug enhancing the level of the Legl protein or enhancing the activity of the Legl protein to activate an Akt signal so as to stimulate the transportation of GLUT2 to a surface of a cell membrane, with the Legl protein as the target.

Or the drug is a drug activating an Akt signaling pathway by enhancing the expression level of the Legl gene to make the GLUT2 transported to the surface of the cell membrane.

In the eighth aspect, the present disclosure provides a method for treating obesity or reducing weight, comprising: inhibiting the level of the Legl protein in an individual body, with the Legl protein as the target;
or blocking the binding of the Legl protein to the EGFR protein, with the Legl protein as the target;
or inhibiting the activity of the Legl protein, with the Legl protein as the target;
or inhibiting the expression level of the Legl gene encoding the Legl protein, with the Legl gene as the target;
or silencing the expression of the Legl gene by using an RNA interference vector capable of silencing the Legl gene,
wherein the amino acid sequence of the Legl protein is as set forth in (1), (2) or (3):
(1) SEQ ID NO: 1;
(2) a derivative sequence that is obtained by subjecting the sequence as set forth in SEQ ID NO: 1 to substitution and/or deletion and/or addition of a plurality of amino acid residues and has the same or antagonizing bioactivity as SEQ ID NO: 1;
(3) a derivative sequence that is obtained by subjecting the sequence as set forth in SEQ ID NO: 1 to substitution, deletion or addition of one amino acid residue and has the same or antagonizing bioactivity as SEQ ID NO: 1.

Further, the obesity is caused by overeating and nutrition surplus.

In the ninth aspect, the present disclosure provides a method for treating lipopenia or gaining weight, comprising: enhancing the level of the Legl protein in an individual body, with the Legl protein as the target;
or promoting the binding of the Legl protein to the EGFR protein, with the Legl protein as the target;
or enhancing the activity of the Legl protein, with the Legl protein as the target;
or enhancing the expression level of the Legl gene encoding the Legl protein, with the Legl gene as the target,
wherein the amino acid sequence of the Legl protein is as set forth in (1), (2) or (3):
(1) SEQ ID NO: 1;
(2) a derivative sequence that is obtained by subjecting the sequence as set forth in SEQ ID NO: 1 to substitution and/or deletion and/or addition of a plurality of amino acid residues and has the same bioactivity as SEQ ID NO: 1;
(3) a derivative sequence that is obtained by subjecting the sequence as set forth in SEQ ID NO: 1 to substitution, deletion or addition of one amino acid residue and has the same bioactivity as SEQ ID NO: 1.

In the tenth aspect, the present disclosure provides a method for treating human diabetes, comprising: enhancing the level of the Legl protein in an individual body, with the Legl protein as the target;
or enhancing the activity of the Legl protein to activate an Akt signal so as to stimulate the transportation of GLUT2 to a surface of a cell membrane;
or activating the Akt signaling pathway by enhancing the expression level of the Legl gene encoding the Legl protein, so as to stimulate the transportation of GLUT2 to a surface of a cell membrane,
wherein the amino acid sequence of the Legl protein is as set forth in (1), (2) or (3):
(1) SEQ ID NO: 1;
(2) a derivative sequence that is obtained by subjecting the sequence as set forth in SEQ ID NO: 1 to substitution and/or deletion and/or addition of a plurality of amino acid residues and has the same or antagonizing bioactivity as SEQ ID NO: 1;
(3) a derivative sequence that is obtained by subjecting the sequence as set forth in SEQ ID NO: 1 to substitution, deletion or addition of one amino acid residue and has the same or antagonizing bioactivity as SEQ ID NO: 1.

Further, the individual is a human being.

Further, preferably, the nucleotide sequence of the Legl gene is as set forth in SEQ ID NO: 4, or is a derivative sequence that is obtained by subjecting the sequence as set forth in SEQ ID NO: 4 to substitution and/or deletion and/or addition of one or more bases and has the same or antagonizing functions as SEQ ID NO: 4.

### Advantageous effects of the Legl protein, the Legl gene, uses and drugs thereof provided by the present disclosure are as follows:

In the present disclosure, the human hLeg1 protein (as set forth in SEQ ID NO: 1) and hLeg1 gene (as set forth in SEQ ID NO: 3) on which no study has been conducted in the prior art and the functions of which are unknown are taken as the subjects, and in order to study the functions thereof, very comprehensive investigation has been conducted on the functions of mLeg1 protein (SEQ ID NO: 2) and the coding gene thereof, i.e., mLeg1 gene (as set forth in SEQ ID NO: 4), by approaches in Genetics, Molecular Biology, Biochemistry and Cell Biology, with mLeg1 knockout mice as the model animal.

The study results show: mLeg1 protein can regulate *in vivo* Akt signals via EGFR protein, so as to further regulate *in vivo* lipogenesis, which demonstrates that mLeg1 gene and mLeg1 protein are closely related to *in vivo* lipogenesis (the enhancement of the expression level of mLeg1 gene, the expression level of mLeg1 protein or the content of mLeg1 protein promotes lipogenesis and fat accumulation; and the inhibition of the expression level of mLeg1 gene, the expression level of mLeg1 protein or the content of mLeg1 protein reduces fat accumulation), and further reveals that human hLeg1 gene (as set forth in SEQ ID NO: 3) or hLeg1 protein (as set forth in SEQ ID NO: 1) can be used as a target gene or a target protein in preparation of drugs related to lipogenesis regulation (e.g., a drug related to treating obesity or weight gaining), in a drug for treating lipopenia or gaining weight, in preparing or screening a drug for treating human diabetes, and in preparing a drug for regulating fat accumulation in vertebrates. The research results of the present disclosure provide a brand-new drug target and a new treatment approach and idea for the development of drugs in the fields of treatment or prevention of obesity at a later stage, physical recovery of cancer patients after chemotherapy, treatment of lipopenia, weight gaining, treatment of human diabetes, and detection of salivary gland diseases, etc.

In order to make the objects, features and advantages of the present disclosure clearer and easier to understand, detailed description is made below with preferred embodiments with reference to the drawings.

### Brief Description of Drawings

In order to more clearly illustrate the technical solutions of the embodiments of the present disclosure, brief description is made below on the drawings required to be used in the embodiments. It should be understood that the following drawings only illustrate some of the embodiments of the present disclosure and shall not be regarded as a limit to the scope, and for those of ordinary skills in the art, other related drawings may be obtained from these drawings without inventive effort.

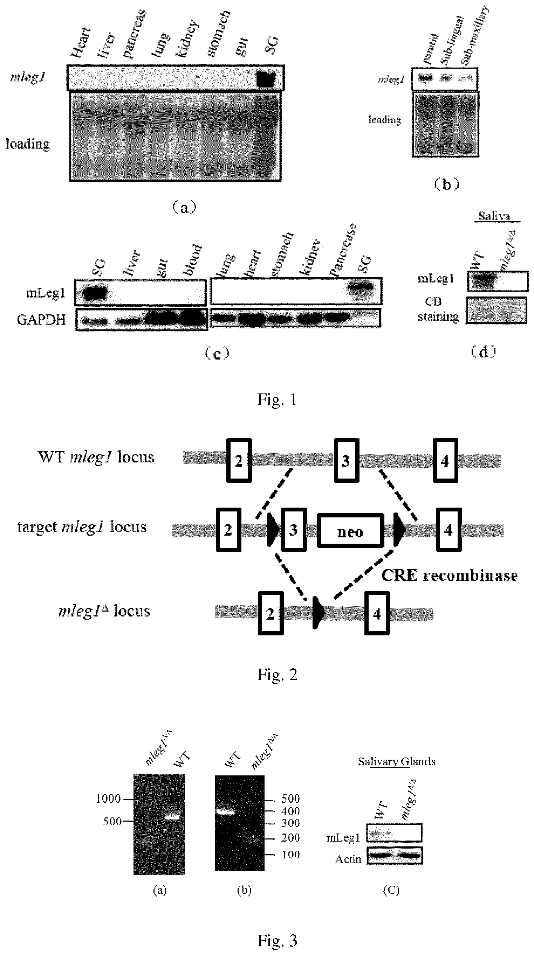

FIG. 1 is a diagram of detection results of Embodiments 1-5 of the present disclosure (in the figure, a is a graph showing the results of expression of mLeg1 in different tissues of a wild-type mouse detected by Northern blot analysis; b is a graph showing the results of expression of mLeg1 in the three glands of salivary glands of a wild-type mouse by Northern blot analysis; c is a graph showing the results of distribution of mLeg1 protein in different tissues of a wild-type mouse detected by Western blot; and d is a graph showing the detection results of the content of mLeg1 protein in saliva of a wild-type mouse and saliva of an mLeg1 knockout mouse);
FIG. 2 is a schematic diagram of mLeg1 knockout strategy in Embodiment 4 of the present disclosure;
FIG. 3 is a diagram of gel electrophoresis detection results of Embodiments 4 and 5 of the present disclosure (in the figure, a is a graph showing gel electrophoresis results of an mLeg1^{Δ/Δ} mouse in which the third exon has been knocked out, detected by a common PCR method; b is a graph showing gel electrophoresis results of an mLeg1^{Δ/Δ} mouse in which the third exon has been knocked out, detected by RT-PCR; and c is a graph showing the results of expression of mLeg1 protein in the salivary glands of mLeg1^{Δ/Δ} mice, detected by the method of Western blot);

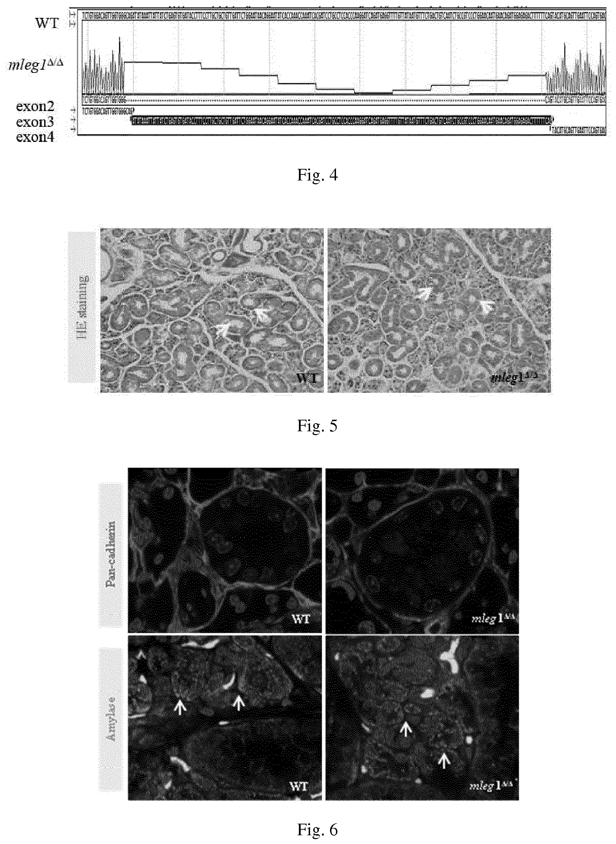

FIG. 4 is a comparison diagram of the sequencing results of mLeg1 gene of the mLeg1^{Δ/Δ} mice in Embodiment 5 of the present disclosure;
FIG. 5 is a comparison diagram of HE staining results between the salivary glands of the mLeg1^{Δ/Δ} mice and the salivary glands of the wild-type mouse in Embodiment 6 of the present disclosure;
FIG. 6 is a comparison diagram of observation results between the salivary glands of the mLeg1^{Δ/Δ} mouse and the salivary glands of the wild-type mouse, obtained by protein immunofluorescent labeling using a salivary amylase and a connexin pan-Cadherin in Embodiment 6 of the present disclosure;

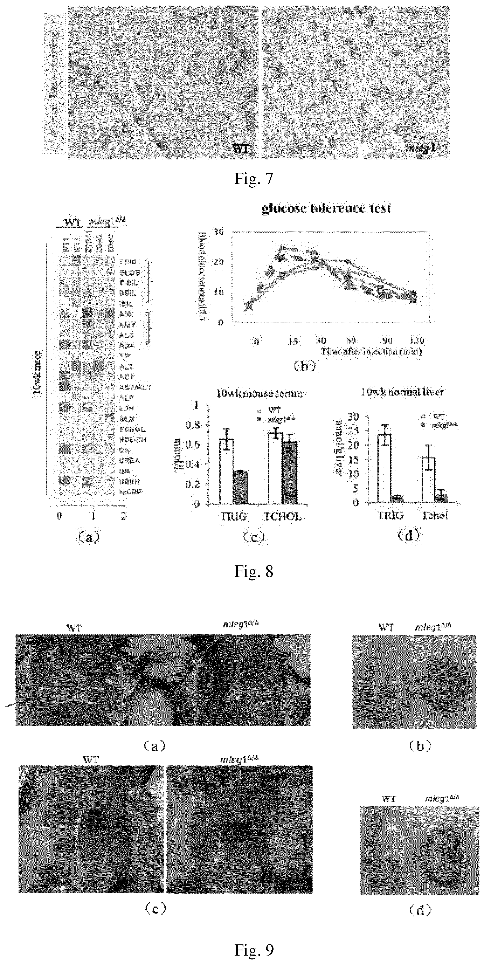

FIG. 7 is a comparison diagram of the detection results of Alcian Blue staining between the mucus secreted by the salivary glands of an mLeg1^{Δ/Δ} mouse and the mucus secreted by the salivary glands of mLeg1 in Embodiment 6 of the present disclosure;
FIG. 8 is a diagram of the detection results of Embodiments 7 and 8 of the present disclosure (in the figure, a is a graph showing the detection results of the blood indexes of an mLeg1^{Δ/Δ} mouse and a wild-type mouse; b is a graph showing the detection results of the glucose tolerance levels of an mLeg1^{Δ/Δ} mouse and a wild-type mouse; c is a graph showing the detection results of the content of triacylglycerol and the content of cholesterol in the serum of an mLeg1^{Δ/Δ} mouse and the serum of a wild-type mouse; and d is a graph showing the detection results of the content of triacylglycerol and the content of cholesterol in the liver of an mLeg1^{Δ/Δ} mouse and the liver of a wild-type mouse);
FIG. 9 is a diagram of size comparison of fats collected from different parts between an mLeg1^{Δ/Δ} mouse and a wild-type mouse in Embodiment 9 of the present disclosure (in the figure, a is an intuitive comparison graph of dorsal fat between an mLeg1^{Δ/Δ} mouse and a wild-type mouse; b is a graph of intuitive size comparison of a dorsal side fat block between an mLeg1^{Δ/Δ} mouse and a wild-type mouse; c is an intuitive comparison graph of abdominal fat between an mLeg1^{Δ/Δ} mouse and a wild-type mouse; and d is an intuitive size comparison graph of abdominal side fat blocks between an mLeg1^{Δ/Δ} mouse and a wild-type mouse);

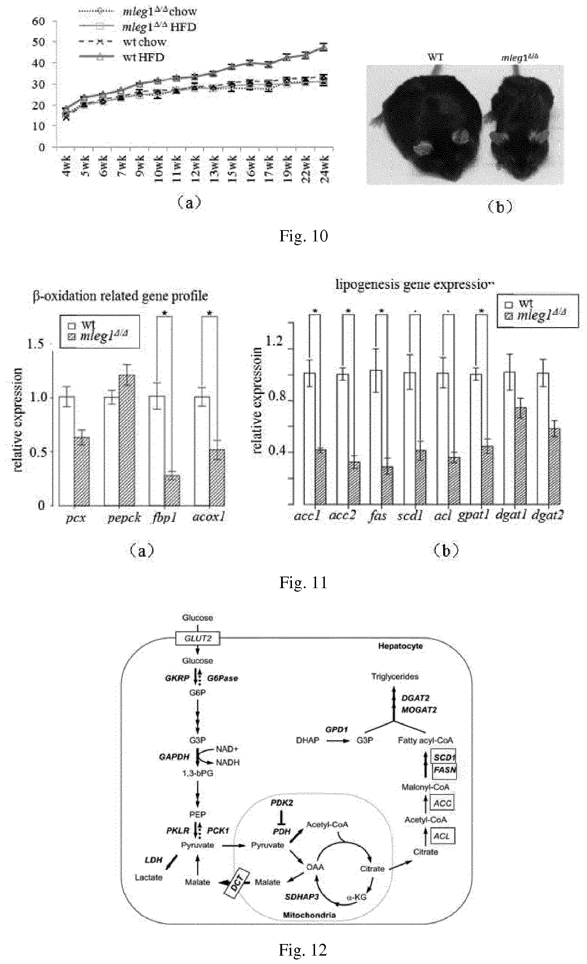

FIG. 10 is a diagram of the detection results of body weight changes of an mLeg1^{Δ/Δ} mouse and a wild-type mouse in Embodiment 9 of the present disclosure (in the figure, a is a body weight change curve of the mLeg1^{Δ/Δ} mice and the wild-type mice under normal feeding and high-fat feeding conditions; and b is a graph of intuitive body size comparison between the mLeg1^{Δ/Δ} mice and the wild-type mice after feeding with high-fat diet for half a year);
FIG. 11 is a diagram of the detection results of the expression level of lipogenesis related genes in Embodiment 9 of the present disclosure (in the figure, a is a graph showing the detection results of the expression level of fatty acid β-oxidation related genes in the liver of an mLeg1^{Δ/Δ} mouse and a wild-type mouse; and b is a graph showing the detection results of the expression level of lipogenesis related genes in the liver of an mLeg1^{Δ/Δ} mouse and a wild-type mouse);
FIG. 12 is a schematic diagram of a synthesis pathway of fatty acid in the liver of a mouse in Embodiment 10 of the present disclosure;

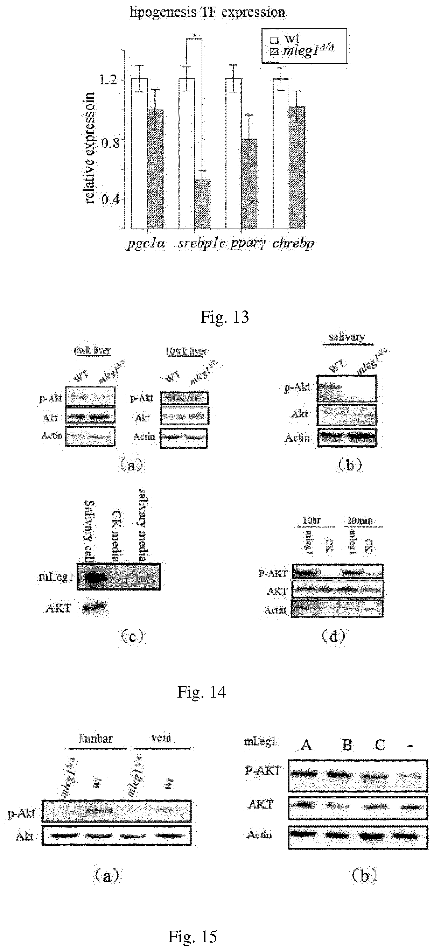

FIG. 13 is a diagram of the detection results of the expression level of a transcription factor regulating lipogenesis in the liver of an mLeg1^{Δ/Δ} mouse and a wild-type mouse in Embodiment 11 of the present disclosure;
FIG. 14 is a diagram of the detection results of Akt phosphorylation level in embodiments 12-14 of the present disclosure (in the figure, a is a graph showing the detection results of Akt phosphorylation level in the liver of an mLeg1^{Δ/Δ} mouse and a wild-type mouse; b is a graph showing the detection results of Akt phosphorylation level in the salivary glands of an mLeg1^{Δ/Δ} mouse and of a wild-type mouse; c is a graph showing mLeg1 protein level in a cell culture solution of the salivary gland cells of a wild-type mouse and an mLeg1^{Δ/Δ} mouse; and d is a graph showing the detection results of Akt phosphorylation level of the HepG2 cells cultured by adding the salivary gland cell culture supernatant of an mLeg1^{Δ/Δ} mouse and of a wild-type mouse);
FIG. 15 is a diagram of the detection results of Akt phosphorylation level in Embodiments 15 and 16 of the present disclosure (in the figure, a is a graph showing the detection results of the level of Akt phosphorylation of the liver of an mLeg1^{Δ/Δ} mouse induced by an abdominal cavity injection or a tail vein injection of salivary primary culture supernatant; and b is a graph showing the detection results of Akt phosphorylation levels in the HepG2 cells activated by mLeg1 protein at different concentrations purified from the salivary glands of a wild-type mouse);

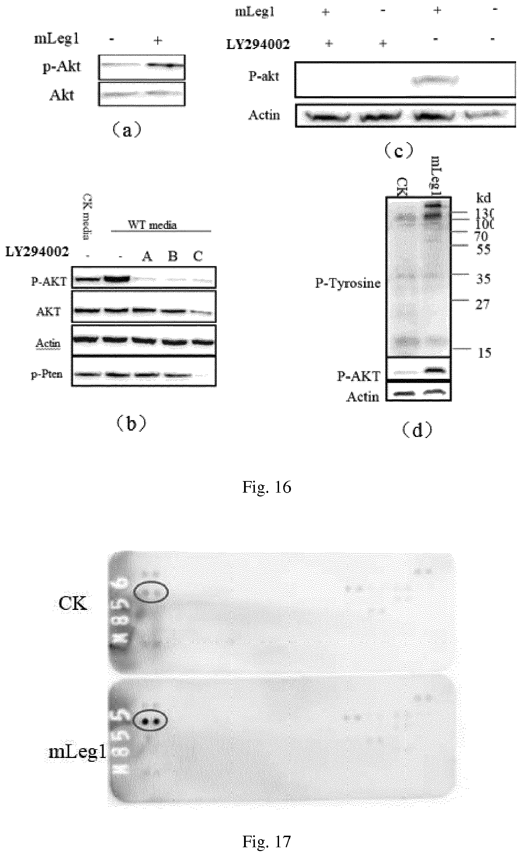

FIG. 16 is a diagram of the detection results of Akt phosphorylation level in Embodiments 16-18 of the present disclosure (in the figure, a is a graph showing the detection results of the level of Akt phosphorylation of the HepG2 cells activated by mLeg1 protein obtained by purification from the salivary glands by column chromatography and ion exchange; b is a graph showing the detection results of the level of Akt phosphorylation of the HepG2 cells cultured by adding the supernatant resulting from primary culture of salivary gland cells from an mLeg1^{Δ/Δ} mouse and a wild-type mouse, under the effect of the inhibitor LY290004; c is a graph showing the detection results of the level of Akt phosphorylation of the HepG2 cells activated by mLeg1 protein obtained by purification from the salivary glands by column chromatography and ion exchange, under the effect of the inhibitor LY290004; and d is a graph showing the detection results of tyrosine phosphorylation level in HepG2 cells cultured by adding partially purified mLeg1 protein;
FIG. 17 is a diagram of screening and detection results of a membrane receptor tyrosine kinase (RTK) in Embodiment 19 of the present disclosure;

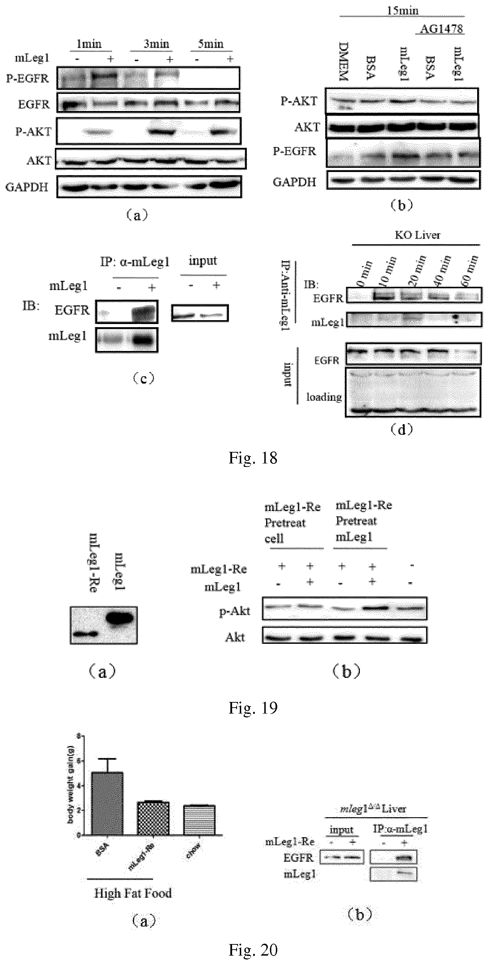

FIG. 18 is a diagram of the detection results according to Embodiments 19-22 of the present disclosure (a is a graph showing the detection results of the activation level of intracellular EGFR protein by mLeg1 protein; b is a graph showing the detection results of the activation level of intracellular EGFR protein by mLeg1 protein under the effect of the inhibitor AG1478; c is a graph showing the detection results of the interaction between mLeg1 protein and EGFR, conducted by the method of co-immunoprecipitation; and d is a graph showing the detection results of the interaction between mLeg1 protein and EGFR in an mLeg1^{Δ/Δ} mouse, intragastrically administered with mLeg1 protein, at different time points);
FIG. 19 is a diagram of the detection results of the recombinant mLegl-Re protein and the detection results of the function thereof according to Embodiment 23 of the present disclosure (in the figure, a is a graph showing the results of molecular weight comparison between the wild-type mLeg1 protein and mLeg1 protein recombinantly expressed by the *Escherichia coli* expression system, detected by the method of immunoblotting; and b is a graph showing the results of Akt phosphorylation of the HepG2 cells after adding mLeg1 protein recombinantly expressed by the *Escherichia coli* expression system;
FIG. 20 is a diagram showing the results of the impact of the recombinant mLeg1 protein on the body weight of a wild-type mouse according to Embodiment 23 of the present disclosure (a is a graph showing the results of the body weight increase of a wild-type mouse intragastrically administered with mLegl-Re protein, under the condition of high-fat-diet feeding; and b is a graph showing the detection results as to whether mLegl-Re protein can reach the liver of mleg1^{Δ/Δ} mice, conducted by the method of co-immunoprecipitation).

### Detailed Description of Embodiments

The technical solutions in the embodiments of the present disclosure will be clearly and completely described below with reference to the drawings of the embodiments of the present disclosure. Obviously, the embodiments described are only some of the embodiments of the present disclosure, rather than all of the embodiments of the present disclosure. Generally, the components in the embodiments of the present disclosure described and illustrated in the drawings herein may be arranged and designed in various different configurations. Thus, the following detailed description of the embodiments of the present disclosure provided in the drawings is not intended to limit the scope of the present disclosure, but only represents the selected embodiments of the present disclosure. All the other embodiments that are obtained by a person skilled in the art on the basis of the embodiments of the present disclosure without any inventive effort shall be covered by the protection scope of the present disclosure.

Below, Legl protein, Legl gene, uses and drugs thereof according to the embodiments of the present disclosure are specifically described.

The inventors of the present disclosure selected mice as the model animals for the study, conducted relevant research on functions of Legl gene (Liver-enriched gene 1, the protein that is encoded to be expressed by the gene is referred to as Legl protein), the homologous gene of the Legl protein in mice, i.e., mLeg1 gene (as set forth in SEQ ID NO: 4), and mLeg1 protein (SEQ ID NO: 2). It revealed that mLeg1 gene and the function thereof in encoding and expressing mLeg1 protein which regulating lipid de novo synthesis, which infers a similar function conferred by the Legl gene and Legl protein in other vertebrates.

mLeg1 is also referred to as 2310057J18Rik RIKEN cDNA 2310057J18 gene (GeneID: 67719), which is a homologous gene of Legl in mice, and the study on the functions thereof in mice is almost blank. The biological information analysis shows that mLeg1 gene is on Chromosome 10, has a total length of about 14.016kb, and comprises six exons and five introns, wherein the ATG initiation code is located at the first exon. mLeg1 gene encodes a protein with a length of 337 amino acids (as set forth in SEQ ID NO: 2). Analytical prediction indicates that the protein contains a leader signal peptide with 20 amino acids, and the sequence of the leader signal peptide is the sequence of the amino acids at Positions 1 to 21 set forth in SEQ ID NO: 2, which indicates that mLeg1 is a novel secretory protein.

The *Homo sapiens* hLeg1 protein (the amino acid sequence thereof is as set forth in SEQ ID NO: 1) and the homologous protein in mice, i.e., mLeg1 protein (the amino acid sequence thereof is as set forth in SEQ ID NO: 2), have a similarity of 71.2%. Therefore, the study of the functions of the coding gene of mice mLeg1 protein, i.e., mLeg1 gene (as set forth in SEQ ID NO: 4), and the functions of mLeg1 protein can provide guidance and reference for the study on the functions and uses of the human hLeg1 gene (the coding sequence is CDS sequence, as set forth in SEQ ID NO: 3) and the hLeg1 protein, and also provide theoretical basis for the development of drugs for fat-related diseases.

There are two copies of the Legl protein in zebrafish (*Danio rerio*)*,* namely dLegla protein (the amino acid sequence is as set forth in SEQ ID NO: 5) and dLeglb protein (the amino acid sequence is as set forth in SEQ ID NO: 6), having a similarity of 47.5% and of 48.6% respectively with mLeg1 protein; oLeg1 protein (the amino acid sequence is as set forth in SEQ ID NO: 7) present in sheep (*Ovisaries*) has a similarity of 49.1% with mLeg1 protein; and bLeg1 protein (the amino acid sequence is as set forth in SEQ ID NO: 8) present in bovine (*Bostaurus*) has a similarity of 45.7% with mLeg1 protein (the method for similarity comparison used in the present disclosure is as follows: comparison is made using the pairing and comparison software of the European Bioinfomatics Institute (EBI), i.e., Needle, with the parameter settings: Matrix: EBLOSUM62, Gap_penalty: 10.0, Extend_penalty: 0.5).

Since the Legl protein is a secretory protein highly conserved in vertebrates, and the Legl proteins in all vertebrates have the same DUF domain (e.g., the amino acid residue sequences at Positions 28-337 in SEQ ID NO: 2, at Positions 28-320 in SEQ ID NO: 1, at Positions 29-362 in SEQ ID NO: 5, at Positions 29-362 in SEQ ID NO: 6, at Positions 34-354 in SEQ ID NO: 7, and at Positions 1-317 in SEQ ID NO: 8 respectively, constitute DUF domains with similar functions in three-dimensional space), these Legl proteins have similar functions and uses prospects. Therefore, uses of the Legl proteins of all the vertebrates and the coding genes thereof as well as uses thereof related to lipogenesis all fall within the protection scope of the present disclosure.

It also needs to be noted that the protein as set forth in a derivative sequence that is obtained by subjecting the sequence as set forth in SEQ ID NO: 1 to substitution and/or deletion and/or addition of one or more amino acid residues and has the same or antagonizing bioactivity as SEQ ID NO: 1, and uses thereof also fall within the protection scope of the present disclosure. As long as the mutant Legl resulting from the above modifications based on the Legl protein as set forth in SEQ ID NO: 1 has the same DUF domain as the Legl protein set forth in SEQ ID NO: 1 and has the same or similar or antagonizing bioactivity as the Legl protein, lipogenesis-related uses of these mutant proteins and the coding genes thereof also fall within the protection scope of the present disclosure.

Similarly, the protein as set forth in a derivative sequence that is obtained by subjecting the sequence as set forth in SEQ ID NO: 2 to substitution and/or deletion and/or addition of one or more amino acid residues and has the same or antagonizing bioactivity as SEQ ID NO: 2, and uses thereof also fall within the protection scope of the present disclosure. As long as the mutant Legl resulting from the above modifications based on the Legl protein as set forth in SEQ ID NO: 2 has the same DUF domain as the Legl protein set forth in SEQ ID NO: 2, which makes the mutant Legl has the same or similar or antagonizing bioactivity as the Legl protein does, lipogenesis-related uses of these mutant proteins and the coding genes thereof also fall within the protection scope of the present disclosure.

It should be noted that vertebrates referred to in the present disclosure not only include human, mice, zebrafish, sheep and bovine, but also include rabbits, pigs, horses, tigers, leopards, wolves, dogs, chicken, ducks, fish, geese, bears, monkeys, etc., and also are not limited thereto.

The present disclosure comprehensively and systematically studies the functions of the novel secretory protein mLeg1 by approaches in Genetics, Molecular Biology, Biochemistry and Cell Biology with the model animal mice and human cell lines as the study models demonstrates that the secretory protein mLeg1 is a novel signaling molecule by providing a large amount of evidence, establishes a signal regulation network from mLeg1 to EGFR/PI3K and finally activating Akt and Srebplc, and demonstrates that the network promotes *in vivo* lipogenesis of the mice. Moreover, the inventors of the present disclosure proved that the mLeg1 knockout mice can grow normally, and more importantly, the mLeg1 knockout mice can resist high-fat diet induced obesity.

The features and properties of the present disclosure are further described in detail below with reference to the embodiments.

### Embodiment 1

### Experimental animals and feeding

Experimental animals: wild-type mice under the C57BL/6 background were selected; and mLeg1 knockout mice (mLeg1^{Δ/Δ} knockout mice) were obtained by using the Cre-loxP system and knocking out mLeg1 gene from the whole body of Cre tool mice of C57BL/6-Tg(Zp3-cre)93Knw/Jnju (all the involved strains of mice were purchased from Nanjing Biomedical Research Institute (NRI)). Feeding conditions: the temperature was 22°C, the humidity was 50%-60%, and a light cycle of 12h light/12h dark was given. The standard chow diet for the mice is irradiated feed (M02-F) for rats and mice produced by Shanghai SLAC Laboratory Animal Co., Ltd., and the high-fat feed is high-fat experimental feed (M04-F) for rats and mice produced by Shanghai SLAC Laboratory Animal Co., Ltd.

### 1. The expression of mLeg1 in different tissues analyzed by Northern blot

The expression spectrum of mLeg1 gene was detected by Northern blot analysis, with 8-week-old male mice under the C57BL/6 background as the subjects. With the antisense strand of mLeg1 gene as a probe, Northern blot analysis was conducted to analyze the expression of mLeg1 gene in a series of organs (heart, liver, pancreas, lung, kidney, stomach, gut and salivary glands (SG)) of the mice including liver.

The experimental method of Northern blot analysis is as follows.

### 1.1 RNA extraction:

1.1.1 The tissue whose RNA was needed to be extracted was ground with liquid nitrogen until no obviously visible particles were left, the entire grinding process was carried out in the presence of liquid nitrogen in order to prevent degradation of RNA.
1.1.2 50-100 mg of the sample was added to 1 ml Trizol (TRIZOL® Reagent, Life Technologies, Cat. No. 15596-026), and was thoroughly homogenized by pipetting through a 26 G needle repeatedly.
1.1.3 The resultant mixture stood at room temperature for 5 minutes, was added with 0.2 ml of chloroform and mixed uniformly for 30 seconds, then was stood for 5 minutes at room temperature, and then was centrifuged at 12000 g at 4°C for 15 minutes to separate the liquid phases from each other.
1.1.4 The aqueous phase (i.e., the liquid in the upmost layer) was added into 0.5 ml of isopropanol, the mixture was inverted and mixed uniformly for incubation for 10 minutes at room temperature to precipitate RNA. The resultant mixture was centrifuged at 12000 g at 4°C for 15 minutes, and the supernatant was removed.
1.1.5 The precipitate was washed by adding 1 ml of 75% ethanol (prepared with DEPC water) thereto, and was centrifuged at 12000 g at 4°C for 5 minutes, and the supernatant was removed. The precipitate was washed with 75% ethanol for a second time, and the supernatant was removed sufficiently. The resultant product was dried at 42°C and then dissolved with a proper amount of DEPC water. The extracted RNA was immediately used for the subsequent experiments or stored in a -80°C refrigerator for later use.

### 1.2 The preparation of digoxin (DIG)-labeled probe:

(1) By using DIG-labeled dNTP (10X PCR DIG Labeling Mix, Roche Cat. No. 11585550910) instead of dNTP, DIG was incorporated into double-stranded DNA by virtue of PCR reaction to serve as a probe for Northern blot. The PCR primers were: probe F: GGCTGTCCTGGCTTCCTG; probe R: CTCTCCATCTGTTCATTGTTCC. The PCR used a common Taq enzyme (reaction system: 1 µl of template, 0.3 µl of positive primers and 0.3 µl of reverse primers, 0.3 µl of Taq enzyme, 2 µl of 10x buffer, 1 µl of 2.5 mM dNTP and 15.1 µl of water) (the Taq enzyme reaction system in the following description is the same as this one), reaction procedures: step 1: 94°C for 3 minutes; step 2: 94°C for 30 seconds; step 3: 58°C for 30 seconds; step 4: 72°C for 30 seconds; step 5: repeating steps 2 to 4 for 33 times; and step 6: 72°C for 10 minutes.
(2) The PCR reaction product was detected in terms of size and purity by agarose gel electrophoresis, and was purified by a PCR purification kit. The purified probe was denatured at 100°C for 10 minutes, then immediately cooled on ice for at least 2 minutes, diluted to 25 ng/ml using a DIG Easy Hyb (Roche Cat. No. 11603558001), and stored at -20°C for later use.

1.3 The preparation of RNA denaturing gel: The RNA gel electrophoresis was carried out in a denaturing buffer and a gel. 10X MOPS buffer (0.2 M MOPS, 50 mM NaOAc, 10 mM EDTA, pH 7.0) was diluted to IX by sterilized deionized water, added with 1.3% agarose powder, fully dissolved by heating by a microwave oven, and added with 5.3% of formaldehyde at the concentration of 37% when cooled to about 50°C, which was then mixed uniformly and poured into a gel-making mould, and left for cooling and solidification for later use.

1.4 The treatment of the RNA sample: A proper amount of RNA (namely the RNA sample extracted in step 1.1, 10-30µg) was added to 17.5 µl of RNA denaturant (containing 10 µl of deionized formamide, 2 µl of 10X MOPS, 3.5 µl of 37% formaldehyde, 2 µl of RNA loading buffer (Ambion® Gel Loading Buffer II, Life Technologies, Cat. No. AM8546G)), denatured at 65°C for 20 minutes, and then immediately placed on ice for 10 minutes.

1.5 RNA denaturing gel electrophoresis: The cooled and solidified RNA denaturing gel was placed in IX MOPS electrophoresis buffer, the RNA sample was loaded thereto for electrophoresis, and an RNA molecular Marker (Fermentas Cat. No. SM1821) was also added thereto for molecular weight estimation, the resultant product was electrophoresed using a voltage of 4-10 V/CM, and the electrophoresis time was determined according to the size of the fragment, which is generally 4-7 hours.

### 1.6 RNA transfer:

1.6.1 The gel (RNA gel) which had undergone the process of RNA denaturing gel electrophoresis was taken out, and was washed with sterilized deionized water and placed in 10X SSC to be balanced. Hybond-N+ membrane (Amersham Bioscience Cat. No. RPN303B) and 3 mm filter paper having a suitable size were tailored according to the size of the gel, which were also balanced in 10X SSC.
1.6.2 10X SSC buffer was poured into a clean open porcelain dish container, and the porcelain dish was covered by a piece of organic glass. Two layers of 3 mm filter paper having a length slightly larger than that of the organic glass and a width slightly larger than that of the RNA gel were tailored to cover the organic glass after being wetted by 10X SSC, with the longitudinal two ends of the filter paper infiltrated in the SSC buffer in the porcelain dish. The RNA gel was upended on the filter paper, and then covered successively with the Hybond-N⁺ membrane, the two-layered 3 mm filter paper and multilayered water absorbent paper, onto which a weight was finally pressed for transferring overnight. After the transferring had been completed, the membrane was taken down and subjected to energy crosslinking at 150 mJ/cm² in an ultraviolet crosslinker (UVP Ultraviolet Crosslinker Cat. No. CL-1000), followed by staining with RNA methylene blue staining solution (0.3 M NaOAc, pH 5.2, 0.03% Methylene Blue), to detect the result and quality of the RNA transferring.

### 1.7 Probe hybridization and development analysis:

The hybridization and cleaning of the DIG probe were carried out by the DIG washing and blocking kit (Roche Cat. No. 11585762001) from the Roche company according to the manufacturer's instruction, which were specifically as follows:
1.7.1 The RNA membrane (resulting from step 1.6.2) was placed in a hybridization tube, and a suitable amount of pre-hybridization solution (Roche Cat. No. 11603558001) was added thereto for blocking at 50°C for 2 hours, during the process, the DIG-labeled probe stored at -20°C was taken out to be denatured at 100°C for 10 minutes and then balanced at 50°C. After the 2 hours' blocking, the balanced probe was added for hybridization at 50°C overnight.
1.7.2 Next day, the probe was recovered, and the RNA membrane was washed in an order as follows: washing once at normal temperature using 2X SSC/0.1% SDS, 10 minutes; washing twice at 65°C using 0.5X SSC/0.1% SDS, 15 minutes each time; washing twice at 65°C using 0.1X SSC/0.1% SDS, 15 minutes each time; and washing at room temperature for 10 minutes using the washing buffer. The resultant product was added with 10% DIG blocking buffer and blocked for 1 hour, and then was incubated at room temperature for 2 hours using the Anti-Digoxigenin-AP Fab fragments antibody (Roche Cat. No. 11093274910) diluted with 10% DIG blocking buffer at 1:20000. The product was then washed twice with the washing buffer, 15 minutes each time.
1.7.3 Finally, the membrane was balanced for 5 minutes using a detection butter. The membrane was clamped in a plastic membrane, added dropwise with Ready-to-use CDP-star solution (Roche Cat. No. 12041677001) for developing, and then imaged in a fluorescent chemiluminescent imager (Clinx Science Instruments Cat. No. 3400). The results were as shown in FIG. 1(a).

As could be known from FIG. 1(a) (in FIG. 1(a), SG represents salivary glands, liver represents liver, gut represents gut, lung represents lung, heart represents heart, stomach represents stomach, kidney represents kidney, and pancreas represents pancreas), mLeg1 gene was not enriched-expressed in liver, but had very high expression in salivary glands (SG), and substantially no expression of mLeg1 was detected in other tissues (heat, liver, pancreas, lung, kidney, stomach and gut).

The salivary glands of a mouse mainly comprise three parts, namely submandibular gland, sublingular gland and parotid. Therefore, by using Northern blot analysis (the specific method is the same as that in Embodiment 1), the inventors of the present disclosure studied the expression of mLeg1 gene in the three glands, and the results were as shown in FIG. 1(b).

As could be known from in FIG. 1(b) (in FIG. 1(b), parotid represents parotid, sub-lingual represents sublingular gland, and sub-maxillary represents submandibular gland), mLeg1 gene was significantly expressed in each of the three glands, and the expression thereof in the parotid tissue was higher than the expression thereof in submandibular gland and in sublingular gland.

### Embodiment 2

The homologous protein Legl of mLeg1 protein in zebrafish is a secretory protein, and the above results have already showed that mLeg1 gene is mainly expressed in the salivary glands, but mLeg1 protein thereof may be secreted and transported to other tissues to function. Therefore, the inventors of the present disclosure extracted the total protein of different tissues of the mice, and detected the distribution conditions of mLeg1 protein in different tissues by Western blot.

The distribution situations of mLeg1 protein in different tissues were detected by Western blot. The experimental method was as follows:

### 2.1 Protein extraction:

After sudden death of the mice, target tissues (SG, liver, gut, blood, lung, heart, stomach, kidney and pancreas) were harvested and were respectively placed in a 1.5 ml centrifuge tube and rapidly frozen in liquid nitrogen so as to prevent degradation. At the time of extracting protein, the sample was taken out and ground through liquid nitrogen, and the sample powder was collected in a centrifuge tube, added with a protein lysate (150 mM NaCl, 50 mM pH 7.6 Tris-Hcl, 0.1% SDS, 1% Triton X100, 1.5% sodium deoxycholate, IX Complete(EDTA-free) (Roche Cat. No. 11873580001), at 100 µl of lysate per 100 mg of sample), and placed on ice, and then was subjected to pipetting through a 26G needle repeatedly for several times, incubation for 15 minutes at 4°C in a vertical shaker, and centrifugation at 12000 g at 4°C for 15 minutes, and the supernatant was collected and the protein concentration thereof was measured through the Braford method.

### 2.2 Immunoblotting (Western blot):

2.2.1 10-20µg of the prepared protein sample was subjected to SDS-PAGE gel electrophoresis, and the protein in the gel was transferred to a PVDF membrane (Millipore Cat. No. IPVH00010) by virtue of a semi-dry transferring instrument (TRANS BLOT® SD SEMI-DRY TRANSFER CELL (Bio-Rad Cat. No. 170-390). The transferring conditions were: 20V and 140 mA, and the transferring time was determined according to the size of the protein, which was generally between 50 minutes and 60 minutes.
2.2.2 After transferring, blocking was carried out for 1 hour with 5% skimmed milk, and then the target protein antibody (which was determined according to the detected target protein, and was an mLeg1 antibody in the present embodiment, and the dilution ratio of which was determined according to the antibody, and was generally 1:1000) diluted in milk was added thereto for incubation at room temperature for 1 hour or at 4°C overnight.
2.2.3 The resultant product was washed with PBST (0.1% Tween 20 in PBS) at 100-150rpm for 5x5 minutes, and added with the corresponding secondary antibody (horseradish peroxidase labeled goat anti-mouse IgG (Beyotime Cat. No. A0216) or horseradish peroxidase labeled goat anti-rabbit IgG (Beyotime Cat. No. A0208)) diluted in milk at 1: 10000 for incubation for 1 hour at room temperature, and then was washed with PBST at 100-150rpm for 5x5 minutes.
2.2.4 The resultant product was added with substrate (Thermo Cat. No.34095), and was imaged in the fluorescent chemiluminescent imager (Clinx Science Instruments Cat. No. 3400). The results were as shown in FIG. 1 (c).

As could be known from FIG. 1 (c) (in FIG. 1 (c), SG represents salivary glands, liver represents liver, gut represents gut, blood represents serum, lung represents lung, heart represents heart, stomach represents stomach, kidney represents kidney, and pancreas represents pancreas), mLeg1 protein was mainly present in the salivary glands (SG), and no significant presence of mLeg1 protein was detected in the other tissues including the liver, the gut, the lung, the heart, the stomach, the kidney and the pancreas. Moreover, there was no massive mLeg1 in the blood of the mice. Thus, for the mice, *in vivo* protein synthesis and storage of mLeg1 mainly occurred in the salivary glands.

### Embodiment 3

The salivary glands are a secretory gland, and the most important function thereof is to secrete saliva. mLeg1 is also a secretory protein. Accordingly, the inventors of the present disclosure studied whether the mLeg1 can be secreted into saliva or not.

The content of mLeg1 in saliva was detected by Western blot. The specific steps were as follows:
3.1 Collection of saliva: Pilocarpine (Pilocarpine, Sigma) was injected at 0.5mg/kg into the abdominal cavity of a mouse, and a capillary tube was placed in the oral cavity of the mouse to guide and collect the secreted saliva. Pilocarpine is a drug for treating oral cavity dryness, and is capable of promoting the secretion of a large amount of saliva. The saliva secreted by the wild-type mice and by the mLeg1 knockout mice (as to the method for obtaining the mLeg1 knockout mice, reference can be made to the text below) was collected separately.
3.2 Treatment of saliva: The collected saliva was boiled for 5 minutes at 100°C using 5x Laemmli buffer (10% SDS, 250 mMTris-HCl, 0.1 ‰ Bromphenol blue, 500 mM DTT, 50% Glycerol) in a volume 1/5 that of the saliva.
3.3 The content of mLeg1 protein in the saliva was analyzed by Western blot, and as to the specific operations, reference can be made to the Western blot detection steps in Embodiment 2. The results were as shown in FIG. 1(d).

As could be known in FIG. 1(d), (in FIG. 1(d), WT represents wild-type mice, and mLeg1^{Δ/Δ} represents mLeg1 knockout mice), there was indeed massive mLeg1 protein in the saliva of the wild-type mice, while no mLeg1 protein was present in the saliva of the mLeg1 knockout mice.

### Embodiment 4

### Obtaining of mLeg1 knockout mice (mLeg1^{Δ/Δ})

In order to obtain the mLeg1 knockout mice, the inventors of the present disclosure knocked out mLeg1 gene from the mice by using the classic Cre-loxP system. This system mainly relies on the mechanism that Cre enzyme can identify the loxP sequence, and delete the sequences from the two loxP sequences in the same direction, thereby achieving the purpose of gene knockout. When the Cre enzyme is expressed in specific time and space, the mLeg1 can be knocked out in specific time and space, thereby avoiding the study difficulty caused by the lethality of the embryos. Here, the inventors of the present disclosure inserted the loxP sequences on either side of the third exon of the mLeg1, and at the same time, added one NEO gene between the third exon and the following loxP sequence for forward resistance screening. By means of homologous recombination, embryo transplantation and genetic screening, the inventors of the present disclosure obtained mleg^{fl/fl} stably inherited transgenic mice in which the loxP sequences had been added to the two ends of the third exon of the mLeg1.

Another part constituting the Cre-loxP system is Cre enzyme. When driven to be expressed by a promoter activated in specific space or at specific time, the Cre enzyme can delete the loxP sequence in specific space or time. The knocking-out strategy for obtaining the mLeg1 knockout mice based on the principle is as shown in FIG. 2. Here, the inventors of the present disclosure selected the mice whose Cre expression was driven by a zp3 promoter and knocked out mLeg1 from the whole body of the mice. Zp3 is a zona pellucida (zona pellucida glycoprotein 3) gene which is only expressed in oocytes before initial meiosis.

Therefore, when the mLeg1^{fl/fl} mice mated with the mice (C57BL/6-Tg(Zp3-cre)93Knw/JNju) whose Cre expression was driven by zp3, Zp3-CRE⁺ mLeg1^{fl/wt} mice were obtained. In the oocytes generated by the female mice therein, the activation of the zp3 promoter induced the expression of the Cre enzyme, thereby knocking out the third exon of mLeg1 gene. By the obtained female mice mating with the wild-type male mice, ZP3-CRE⁺ mLeg1^{Δ/WT} and ZP3-CRE⁻ mLeg1^{Δ/WT} mice were obtained, and mLeg1^{Δ/Δ} and mLeg1^{WT/WT} mice could be obtained by inbreeding of the ZP3-CRE⁻ mLeg1^{Δ/WT} mice. The mLeg1^{Δ/Δ} mice were exactly mLeg1 knockout mice.

Identification of the mLeg1^{Δ/Δ} mice from the mLeg1^{Δ/Δ} and mLeg1^{WT/WT} mice by the PCR method:
1mm tail were scissored off the mice and were numbered, and the scissored tail were collected and genome DNA was extracted by the method of alkaline lysis. 75 µl of lysate I (25 mM NaOH, EDTA 0.2 mM, pH 12) was added to the collected tail, and the mixture was kept at 95°C for 30 minutes, and then cooled on ice. Further, 75 µl of lysate II (Tris 40 mM, pH 5) was added for neutralization. After adequate reaction, the resultant product was used as a PCR template, and PCR reaction was conducted by the addition of the template at 4 µl of template per 20 µl of PCR reactants. The gene-type identification primers: an upstream primer mLeg1 Fwd: CCTTTCTTAATGACACTTCAGTATGT, and a downstream primer mLeg1 Rv: CACATGCC-TATTCACTCTCTCC. Common Taq enzyme was used for PCR, and the reaction conditions were as follows: 1. 94°C for 3 minutes; 2. 94°C for 30 seconds; 3. 58°C for 30 seconds; 4. 72°C for 30 seconds; 5. repeating steps 2-4 for 33 times; and 6. 72°C for 10 minutes. The PCR product was subjected to a gel electrophoresis experiment, wherein the wild-type mice produced an 685bpband, and the mutant mice produced a 293bp band due to the deletion of the third exon and some of the introns (as shown in FIG. 3(a)).

The identified mLeg1^{Δ/Δ} mice were fed normally for later experiments.

In addition, the results of the hybridization showed: when inbreeding was carried out among the mLeg1^{Δ/w} mice, the mLeg1^{Δ/Δ} mice could be normally born, exhibiting the normal 1:3 Mendelian inheritance ratio; the infant mice could grow into healthy adult mice, and the mLeg1^{Δ/Δ} adult mice could normally produce offspring, and the number of infant mice per litter was not significantly different from that of the wild type.

### Embodiment 5

### Verification of the mLeg1^{Δ/Δ} mice

In order to further verified that mLeg1 had been knocked out, the inventors of the present disclosure collected the salivary glands of the mice that had been identified as mLeg1^{Δ/Δ} mice and the salivary glands of the mice that had been identified as mLeg1^{WT/WT} mice, and extracted the total RNA and synthesize cDNA. The experimental method was as follows.
5.1 Extraction of total RNA: The total RNA of the salivary glands of the mLeg1^{Δ/Δ} mice and of the mLeg1^{WT/WT} mice was extracted separately by the same method used for RNA extraction in step 1.1 in Embodiment 1.
5.2 Reverse transcription of RNA into cDNA: 1 µl of 50µM OligodT and then 1 µl of 10 mM dNTP were added to 1 µl of the extracted RNA sample, and then RNase-free water was added thereto until the total volume reached 10 µl. The mixture was denatured at 65°C for 5 minutes, and then placed on ice for at least 1 minute. 10 µl of cDNA mixture (4 µl 5x First Line Buffer, 2 µl 0.1M DTT, 1 µl M-MLVRT enzyme, 3 µl DEPC water) was added thereto for reaction at 37°C for 50 minutes, and the reaction was kept on until the temperature reached 70°C for 15 minutes, and then was terminated. The synthesized cDNA was used for the subsequent experiments or stored in a -20°C refrigerator.

### 5.3 Identification of PCR:

The cDNA of the wild-type mice and of the mLeg1 knockout mice was subjected to PCR by using the primers 2qPCR F282: CCTCTGCAGTTTGGCTGGCAGT and 3'ARM rev-1: TCCAAGGATGAGGCATGGGCTTC on either side of the third exon of mLeg1 gene, respectively. Common Taq enzyme was used for the PCR, and the reaction conditions were: 1. 94°C for 3 minutes; 2. 94°C for 30 seconds; 3. 58°C for 30 seconds; 4. 72°C for 30 seconds; 5. repeating steps 2-4 for 33 times; and 6. 72°C for 10 minutes.
5.4 The amplified product was subjected to gel electrophoresis, and the results were as shown in FIG. 3(b) (in FIG. 3(b), WT represents the wild-type mice, and mLeg1^{Δ/Δ} represents mLeg1 knockout mice), the wild-type mice produced a band of about 377bp, and the mutant mice produced a band of 192bp due to the deletion of the third exon. Moreover, the amplified product was purified and then sequenced, and the sequencing results were as shown in FIG. 9, in which it had been demonstrated that the third exon of the PCR product of mLeg1^{Δ/Δ} had been deleted (as shown in FIG. 4).
5.5 The level of mLeg1 protein in the salivary glands of the mLeg1^{Δ/Δ} mice and of the wild-type mice was detected by Western blot, and as to the specific steps, reference can be made to Embodiment 2. The detection results were as shown in FIG. 3(c).

As could be known from FIG. 3(c), mLeg1 protein was indeed not detected in the salivary glands of the mLeg1^{Δ/Δ} mice, but was detected in the salivary glands of the wild-type mice.

The above data fully demonstrated that the inventors of the present disclosure had obtained the mLeg1^{Δ/Δ} mice, and the knockout of mLeg1 gene did not influence the survival and propagation of the mice. Thus, the results of the study on the functions of mLeg1 gene with the mLeg1^{Δ/Δ} mice as the model animal would be of high reliability.

### Embodiment 6

The impacts of the knockout of mLeg1 gene on the structure and functions of the salivary glands were investigated.

mLeg1 is expressed in each of the three glands of the salivary glands of a mouse, and submandibular gland is the biggest part of the salivary glands of the mouse. Accordingly, the inventors of the present disclosure took submandibular gland as the subject to study whether the knockout of mLeg1 gene would influence the structure and the functions of the submandibular gland.
6.1 Whether the knockout of mLeg1 gene would influence the morphological structure of the salivary glands was examined using the HE staining method.
   6.1.1 Preparation of a submandibular gland tissue section: The mouse submandibular gland was fixed for 1 hour at room temperature using 4% paraformaldehyde (Sigma, Cat. No. P6148, dissolved in PBS), then washed twice with PBS, 10 minutes each time before being embedded with a 1.5% low-melting-point agarose solution (formulated by boiling and dissolving a 30% sucrose-PBS solution, keep at 45 °C) having a temperature of about 45°C in a small space, e.g., in a cap of a 1.5 ml Eppendorf tube, and then was balanced overnight at 4°C in a 30% sucrose-PBS solution. After balancing, these small blocks were fixed at the bottom of a plastic model using an O.C.T. compound (Sakura Cat. No.: 4583). Then the plastic model was placed in -80°C pre-cooled alcohol supplied with dry ice to frozen. The frozen samples were immediately used, or stored in a sealed box at -80°C. At the time of cryosectioning, the frozen sample block was fixed in a support by using an O.C.T. compound. After being balanced at -30°C for two hours in a microtome (Leica, HM505), the sample was cut into slices with the thickness of 8-12µm. The cut slices were collected on polylysine coated glass slides (Menzel, Cat. No.: J2800AMNZ), and the collected samples were immediately used or stored at -80°C.
   6.1.2 HE staining: The frozen sections were taken out and subjected to hematoxylin staining for 5 minutes, washing with running water for 5 minutes, differentiation with 1% HCl-ethanol (1% of hydrochloric acid + 99% of ethanol) for 5 seconds, washing with running water for 10 minutes, and eosin staining for 5 minutes, and then washing with 80%, 95% and 100% ethanol sequentially, each for 2 seconds to wash off eosin. The resultant product was transparentized by adding xylene thereto, sealed by adding Canada balsam dropwise thereto, and then observed by microscope. The results were as shown in FIG. 5.

As could be known from FIG. 5, there was no significant difference in the HE staining results between the salivary glands of the mLeg1^{Δ/Δ} mice and the salivary glands of the wild-type mice. Both of them had hollow and deeply eosin-stained ducts, and lightly eosin-stained substantive acinar tissues, and they both had a complete and compact structure, suggesting that the knockout of mLeg1 had no significant impact on the development and morphological structure of the ductal transportation system and the saliva secretion unit of the salivary glands.
6.2 Whether the knockout of mLeg1 gene would influence the morphological structure of the salivary glands was examined using the protein immunofluorescence labeling method.
   In order to further determine whether the knockout of mLeg1 would influence the morphological structure of the salivary glands, the inventors of the present disclosure selected two salivary markers, amylases and pan-cadherin to stain the salivary glands, so as to analyze and study the impact of the mLeg1 knockout on the morphological structure of the salivary glands at the cellular level. The experimental method was as follows.
   6.2.1 Preparation of a submandibular gland tissue section: The method was the same as that in step 6.1.1, or the tissue sections prepared in step 6.1.1 were directly used.
   6.2.2 The above treated tissue sections were permeated with PBST (0.2% triton X100) to improve the permeability of the membrane so as to make it easy for an antibody to pass through the cell membrane. The treatment time was generally 5 minutes, and then the sections were washed for 10 minutes using PBB (0.5%BSA (Sangon Cat. No. A0332) dissolved in 1×PBS).
   6.2.3 The sections were blocked using 20% goat serum formulated by PBB, and incubated with primary antibody, namely pan-cadherin antibody (Sigma C1821, 1:100) overnight at 4°C. The sample was then washed with PBB at 60rpm for 3x10 minutes. Fluorescent secondary antibody (Goat anti-Mouse IgG (H+L) Secondary Antibody, Alexa Fluor Plus 647, Thermo, A32728) and DAPI (Beyotime Cat. No. C1002) was diluted by PBB at a ratio of 1:400 and 1:500. before being used to incubate the sample for 1 hour at room temperature. The sample was then washed with PBB at 60rpm for 3x10 minutes, followed by mounting with 80% glycerol.
   6.2.4 Data was collected by a confocal microscope (Olympus FV1000). The results were as shown in FIG. 7.
   6.2.5 Amylase protein immunofluorescent labeling was performed. The method thereof is substantially the same as that in steps 6.2.1-6.2.4, and the differences lie in: in step 6.2.3, the pan-cadherin antibody was replaced with Amylase antibody (anti-salivary amylase antibody, Santa Cruz sc-9890), and the fluorescent secondary antibody was replaced with Goat anti-Rabbit IgG (H+L) Secondary Antibody (Alexa Fluor 488, Thermo, A-11034). The results were as shown in FIG. 6.

As could be known from FIG. 6, the knockout of mLeg1 did not influence the expression and distribution of the salivary amylases (Amylase) and pan-cadherin, suggesting that the knockout of mLeg1 indeed does not significantly influence the tissue structure, cell composition and distribution of the salivary gland cells.
6.4 The saliva production function of the salivary glands of the mLeg1^{Δ/Δ} mice was studied.
   An important function of the salivary glands is to produce and secrete saliva, and therefore, the inventors of the present disclosure also studied the saliva production function of the salivary glands of the mLeg1^{Δ/Δ} mice. The mucus (mucin) secreted by the acinar cells can be stained by Alcian Blue. Therefore, the inventors of the present disclosure evaluated the secretion ability of the salivary glands by Alcian Blue staining. The submandibular gland sections of the wild-type mice and the mLeg1^{Δ/Δ} mice were respectively stained by Alcian Blue. The method was as follows.
   6.4.1 Preparation of submandibular gland tissue sections: The preparation step thereof was the same as step 6.1.1.
   6.4.2 Alcian Blue staining: The sections were rehydrated using double-distilled water, treated for 3 minutes using 3% acetic acid, stained by an Alcian Blue staining solution (1% Alcian Blue, 3% glacial acetic acid, pH 2.5) for 30 minutes at room temperature, washed with running water for 2 minutes, rinsed with double-distilled water, then rinsed with xylene and dehydrated, and finally mounted with Canada balsam for microscopic observation. The results were as shown in FIG. 7.

As could be known from the results shown in FIG. 7, the acini between the ducts of the wild-type mouse and of the mLeg1^{Δ/Δ} mice all had very significant concentrated Alcian Blue positive signals (the positions indicated by the arrows in FIG. 7), meaning that all the submandibular gland acini can normally produce and secrete mucus. Therefore, the knockout of mLeg1 gene does not influence the submandibular gland's ability to secrete saliva.

### Embodiment 7

7.1 The lipid content in the plasma of the mLeg1^{Δ/Δ} mice was determined.
   Since mLeg1 is a secretory protein, and the knockout of mLeg1 seems to have no influence on the development and functions of the salivary glands of the mice, the salivary glands may not be the target organ of mLeg1, that is, mLeg1 may be transported to other organs to function. In order to study the functions of mLeg1, the inventors of the present disclosure examined the whole body of the mice, and studied whether there would be any physiological abnormalities in the mLeg1^{Δ/Δ} mice. The inventors of the present disclosure drew blood from the mice, and determined various blood indexes in the serum. The experimental method was as follows.
   7.1.1 After the mice were anesthetized, blood was drawn from femoral artery, placed in an anticoagulant tube, and then centrifuged at 1000 g for 5 minutes, and the supernatant was collected.
   7.1.2 The diluted supernatant was subjected to an automatic biochemical analyzer (performed by Dian Diagnostics) (Olympus) for detection of various blood indexes. The detection results were as shown in FIG. 8(a).

As could be known from FIG. 8(a) (in FIG. 8(a), the upper brace indicates the indexes that have been decreased, and the lower brace indicates the indexes of the mLeg1^{Δ/Δ} mice that have been increased, WT1 and WT2 both represent the wild-type mice and ZCBA1, ZGA2 and ZGA3 represent the mLeg1^{Δ/Δ} mice), the content of triacylglycerol in the mLeg1^{Δ/Δ} mice was remarkably reduced. In addition, the three bile acids (T-BIL, DBIL and IBIL) were also reduced. Since bile acid is related to the absorption and metabolism of lipid, this means that the metabolism of the mLeg1^{Δ/Δ} mice, especially the lipid metabolism, may be abnormal. Therefore, the inventors of the present disclosure carried out a classical experiment, namely glucose tolerance test, to determine whether there was a metabolic disorder in the mice.
7.2 Glucose tolerance test: The specific method thereof was as follows.
   7.2.1 Before conducting the test, the mice were starved overnight, so that the blood glucose thereof was reduced to the lowest level, glucose solution (dissolving glucose in the sterilized PBS) was injected by intraperitoneal injection at 1g of glucose per kg of mouse body weight (using 1g of glucose for per kg of the mouse body weight), and the blood glucose content in the mice was detected at 0 minute, 15 minutes, 30 minutes, 60 minutes and 90 minutes after injection. The blood glucose content was detected by the Roche glucometer (ACCU CHEK). The results were as shown in FIG. 8(b).

As could be known from the results shown in FIG. 8(b) (in FIG. 8(b), the solid line represents the mLeg1^{Δ/Δ} mice, the dotted line represents the wild-type mice), the blood glucose of the wild-type mice rose due to the absorption of glucose, and reached the top point 30 minutes after the injection, and thereafter began to drop slowly for the reason that in order to maintain the blood glucose balance of the body, the body will secrete insulin to reduce the blood glucose content. With regard to the mLeg1^{Δ/Δ} mice, glucose was absorbed more rapidly and entered blood circulation, the blood glucose content reached the highest point 10 minutes after injection, which was higher than that of the wild-type mice. Further, the glucose in the blood of the mLeg1^{Δ/Δ} mice returned to the balanced state at a higher speed. Therefore, although the blood glucose absorption and regulation functions were retained for the mLeg1^{Δ/Δ} mice, the glucose metabolism of the mLeg1^{Δ/Δ} mice was somewhat abnormal.

### Embodiment 8

### Detection of the lipid content in the liver of the mLeg1^{Δ/Δ} mice

Liver is the largest organ in a mammal, and is the center of the metabolism of the body, and is an important place for lipid synthesis and catabolism. In addition, when liver synthesizes lipid, the lipid needs to be transported to the adipose tissues through blood circulation, and when the body is in starvation and needs fat, the lipid stored in the adipose tissues needs to be transported to the liver through blood circulation. That the knockout of mLeg1 influences the functions of liver was demonstrated through the detection of the lipid content in the serum of the mice.
8.1 The lipid content in serum was detected. The experimental method was as follows.
   8.1.1 Serum of 10-week-old wild-type mice and serum of 10-week-old mLeg1^{Δ/Δ} mice were collected separately, and subjected to an instrument to detect the content of triacylglycerol and the content of cholesterol therein. This was repeated for three times, and the results were expressed as averages. The results were as shown in FIG. 8(c).

As could be known from FIG. 8(c) (in FIG. 8(c), the grey column represents the mLeg1^{Δ/Δ} mice, the white column represents the wild-type mice, TRIG represents triacylglycerol and TCHOL represents cholesterol), triacylglycerol in the blood of the mLeg1^{Δ/Δ} mice was reduced, which was about only half that of the wild-type mice.

8.2 The lipid content in liver was detected.

The above results suggest that the knockout of mLeg1 influences the functions of the liver. Therefore, the inventors of the present disclosure focused the study on liver, and detected the lipid content in liver. The results were as shown in FIG. 8(d).

As could be known from FIG. 8(d) (in FIG. 8(d), the grey column represents the mLeg1^{Δ/Δ} mice, the white column represents the wild-type mice, TRIG represents triacylglycerol and TCHOL represents cholesterol), in the liver, triacylglycerol was also remarkably reduced, and at the same time, the cholesterol content in the liver of the mLeg1^{Δ/Δ} mice was also remarkably reduced.

### Embodiment 9

The lipid storage in the adipose tissues of the mLeg1 knockout mice was reduced.

The reduction of the lipid content in the blood and liver of the mLeg1^{Δ/Δ} mice motivated the inventors of the present disclosure to study whether the lipid content in the storage sites of fat (namely the adipose tissues) was reduced. The adipose tissues of a mouse mainly comprise abdominal adipose tissues and dorsal adipose tissues.
10.1 The lipid content in the abdominal adipose tissues and the dorsal adipose tissues of the mice was detected, and the experimental method was as follows.
   10.1.1 The sacrificed mice were dissected to collect the abdominal adipose tissue and the dorsal adipose tissue through conventional methods. The results were as shown in FIG. 9.

As could be known from the results shown in FIG. 9(a) and FIG. 9(b), in the 10-week-old mLeg1^{Δ/Δ} mice, the size of the collected dorsal adipose tissues was remarkably reduced, and the size of the collected abdominal adipose tissues was also reduced to a certain extent (as shown in FIG. 9(c) and FIG. 9(d)). Thus, the knockout of mLeg1 indeed had reduced the lipid storage in the adipose tissue.

### 10.2 The growth of the mice under high-fat-diet condition

The above results also further motivated the inventors of the present disclosure to wonder whether the mice were resistant to the high-fat-diet induced obesity. The mice of different types were fed with high-fat-diet continuously. The experimental method was as follows.
10.2.1 Sufficient standard food or high-fat food was provided in the mouse cage, so that the mice could eat freely.
10.2.2 The mice were weighed at different time points (4, 5, 6, 7, 9, 10, 11, 12, 13, 15, 16, 17, 19, 22 and 24 weeks), this was repeated twice, with 3-6 mice in each group each time, and the results were expressed as averages. With the detection time point as the abscissa and the body weight value (the unit being g) as the ordinate, a curve of body weight change of the mice was drawn, and the results were as shown in FIG. 10(a).

As could be known from FIG. 10(a) (in the figure, chow represents standard chow-diet feeding, HFD represents high-fat-diet feeding, mLeg1^{Δ/Δ} chow represents the mLeg1^{Δ/Δ} mice fed with standard chow diet, mLeg1^{Δ/Δ} HFD represents the mLeg1^{Δ/Δ} mice fed with high-fat diet, wt chow represents the wild-type mice fed with standard chow diet, and wt HFD represents the wild-type mice fed with high-fat diet), when the wild-type mice and the mLeg1^{Δ/Δ} mice were fed with standard chow diet, the body weights of the two types of mice grew along with the age; and when the wild-type mice and the mLeg1^{Δ/Δ} mice were fed with high-fat diet instead of standard chow diet, the wild-type mice gained weight rapidly and finally developed obesity, as they obtained too much energy and stored the energy in the form of fat, but the body weight increase of the mutant mice fed with high-fat diet was not significantly different from that of the mice fed with standard chow diet.

In addition, when fed with high-fat diet for six months, the wild-type mice was increased in size, and a very thick layer of fat was formed in the abdomen and the dorsum, exhibiting very significant obesity symptoms, while the mLeg1^{Δ/Δ} mice still kept the size the mice fed with standard chow diet would have (as shown in FIG. 10(b)). These results further prove that the functions of mLeg1 is closely related to lipid metabolism.

### Embodiment 10

The down-regulation of the fatty acid synthesis ability led to reduction in lipids in the mLeg1^{Δ/Δ} mice.

### 10.1 Detection of the expression levels of fatty acid β-oxidation related enzymes in liver

The reduction of lipid content may result from an increase in lipid consumption on the one hand, and on the other hand, may result from a reduction in fatty acid or triacylglyceride synthesis. The catabolism of fatty acid is mainly realized by β-oxidation in liver. Therefore, whether the reduction in lipid content in the mLeg1^{Δ/Δ} mice resulted from an increase in fat consumption, or from a reduction in fatty acid or triacylglyceride synthesis was demonstrated by detecting the expression level of β-oxidation related enzymes by real-time quantitative PCR (qRT-PCR), and the experimental method was as follows.
10.1.1 The expression level of the β-oxidation related enzyme genes (FBP1/PCX/ACOX/PEPCK) in the liver of the wild-type mice and of the mLeg1^{Δ/Δ} mice was detected by using qRT-PCR, with three independent mice in each group, and the amount of gene expression was normalized by taking β-actin as a reference, and then expressed as averages. The qRT-PCR detection method was as follows.
   (1) RNA extraction: The operation method was the same as that in step 1.1 RNA extraction in Embodiment 1, or the RNA sample extracted in step 1.1 RNA extraction in Embodiment 1 was directly detected. (2) RNA purification: Since the total RNA extracted by the method of Trizol may be contaminated by genomic DNA, the RNA sample for fluorescent quantitative PCR was firstly digested by DNA enzyme to remove possible DNA. In a 50 µl reaction system, DNaseI (NEB Cat. No. M0303S) free of RNA enzyme contamination was added to the total RNA at 2 units per 10µg of total RNA, 5 µl of 10x reaction buffer was added thereto, and DEPC water was added thereto to make the total volume 50 µl. The reactants reacted at 37°C for 20 minutes, and the product was subjected to RNA purification using RNeasy® Mini Kit (QIAGEN Cat. NO. 74106). (3) RNA reverse transcription: The purified RNA was reversely transcribed into cDNA through the above steps, and RNA was synthesized into cDNA through the reverse transcription kit (M-MLV First Strand Kit, Life Technologies, Cat. No. C28025-032). (4) The synthesis steps were as follows: 1 µl of 50µM OligodT was added to 1 µl of the extracted RNA sample, 1 µl of 10 mM dNTP was added thereto, and water was added thereto to make the total volume 10 µl, the mixture was denatured at 65°C for 5 minutes, and then placed on ice for at least 1 minute, 10 µl of cDNA mixture (4 µl 5x First Line Buffer, 2 µl 0.1M DTT, 1 µl M-MLVRT enzyme, 3 µl DEPC water) was added thereto for reaction at 37°C for 50 minutes, and after 15 minutes reaction at 70°C, the reaction was terminated. The synthesized cDNA was used for subsequent experiments or stored in a -20°C refrigerator.
   (5) Real-time PCR: The obtained cDNA was used as a template for real-time PCR. The fluorescent quantitative reaction was conducted using SsoFastTM Eva Green® Supermix kit (Bio-Rad Cat. No. 172-5201) according to the manufacturer's instruction. Each reaction was carried out in a 10 µl system including 0.5 µl of cDNA template, 5 µl of Supermix, 0.5 µl of 10µM forward primer, and 0.5 µl of 10µM reverse primer, and 3.5 µl of double-distilled water. The forward and reverse primers for the fluorescent quantitative PCR were: the forward primer: beta actin Fwd: GTGACGTTGACATCCGTAAAGA; and the reverse primer: beta actin Rv: GCCGGACTCATCGTACTCC. The quantification of fluorescence signals was carried out by CFX96TM Real-Time System (Bio-Rad C1000TM Thermal Cycler). The primers used for the genes were as shown in table 1.

The detection results were as shown in FIG. 11(a).

**Table 1 Primer sequence listing for qRT-PCR in the embodiments of the present disclosure**

| **Genes under detection** | **Name of primer** | Primer sequence (5'-3') |
|---|---|---|
| β-actin | β-actin -F | GTGACGTTGACATCCGTAAAGA |
| | β-actin -R | GCCGGACTCATCGTACTCC |
| FBP1 | fbp1-F | TATGGTGGAAAGGGACGGGAA |
| | fbp1-R | CCTCTGGTGATACTCAAGGATGG |
| PCX | pcx-F | CTGAAGTTCCAAACAGTTCGAGG |
| | pcx-R | CGCACGAAACACTCGGATG |
| ACOX | Acox1-F | TAACTTCCTCACTCGAAGCCA |
| | Acox1-R | AGTTCCATGACCCATCTCTGTC |
| PEPCK | Pepck-F | CAAAAACGGCAAGTTCCTCTG |
| | Pepck-F | GACGTAGCCAATGGGAGTGAG |
| ACC1 | Acc1-F | AAGGCTATGTGAAGGATG |
| | Acc1-R | CTGTCTGAAGAGGTTAGG |
| ACC2 | Acc2-F | CTTGCTTCTCTTTCTGACTTG |
| | Acc2-R | GGCTTCCACCTTACTGTTG |
| FAS | Fas-F | GCTGCGGAAACTTCAGGAAAT |
| | Fas-R | AGAGACGTGTCACTCCTGGACTT |
| SCD1 | Scd1-F | GTCAGGAGGGCAGGTTTC |
| | Scd1-R | GAGCGTGGACTTCGGTTC |
| ACL | Acl-F | GCCAGCGGGAGCACATC |
| | Acl-R | CTTTGCAGGTGCCACTTCATC |
| GPAT1 | Gpat-F | CAACACCATCCCCGACATC |
| | Gpat-R | GTGACCTTCGATTATGCGATCA |
| DGAT1 | Dgat1-F | TGGTGTGTGGTGATGCTGATC |
| | Dgat1-R | GCCAGGCGCTTCTCAA |
| DGAT2 | Dgat2-F | AGTGGCAATGCTATCATCATCGT |
| | Dgat2-R | TCTTCTGGACCCATCGGCCCCAGGA |
| SREBP1C | SREBP1C-F | GGAGCCATGGATTGCACATT |
| | SREBP1C-R | GGCCCGGGAAGTCACTGT |
| chrebp | Chrebp-F | CTGGGGACCTAAACAGGAGC |
| | Chrebp-R | GAAGCCACCCTATAGCTCCC |
| PPARr | PPARr-F | GTGCCAGTTTCGATCCGTAGA |
| | PPARr-R | GGCCAGCATCGTGTAGATGA |
| PGC1α | PGC1α-F | TTCATCTGAGTATGGAGTCGCT |
| | PGC1α-R | GGGGGTGAAACCACTTTTGTAA |

As could be known from FIG. 11(a) (in FIG. 11(a), the ordinate represents the relative expression level, and the abscissa represents the relative expression level of the relevant β-oxidase gene), catabolism of fatty acid was mainly realized by β-oxidation in liver, and by comparison of the expression level of β-oxidation related enzymes in liver between the wild-type mice and the mLeg1^{Δ/Δ} mice, it was found that the knockout of mLeg1 gene did not cause any abnormal rise of the expression of these genes, suggesting that the knockout of mLeg1 did not accelerate β-oxidation, i.e., did not cause any increase in the consumption of lipid. The reduction in lipid in the mLeg1^{Δ/Δ} mice probably resulted from a reduction in fatty acid or triacylglyceride synthesis.
10.2 The expression level of fatty acid synthesis related enzymes in liver was determined, and the experimental method was as follows.
   10.2.1 The expression level of fatty acid synthesis related enzymes (ACC1/ACC2/FAS/SCD1/ACL/GPAT1/DGAT1/DGAT2) in the liver of the wild-type mice and of the mLeg^{Δ/Δ} mice was detected using a method similar to that in 10.1.1. The primers used were as shown in table 1.

The detection results were as shown in FIG. 11(b).

As could be known from FIG. 11(b) (in FIG. 11(b), the ordinate represents the relative expression level, and the abscissa represents fatty acid synthesis related enzymes), by comparison of the expression spectrum of fatty acid synthesis related enzyme genes in the liver between the wild-type mice and the mLeg1^{Δ/Δ} mice, it was found that the expressions of the fatty acid *de novo* synthesis related enzyme genes were more or less reduced, some were substantially about half that of the wild-type mice, wherein ACC1, ACC2, FAS and DGAT1 were significantly reduced in the mLeg1^{Δ/Δ} knockout mice.

When further observing the functions of these genes in lipogenesis process, the inventors of the present disclosure found that these genes encoded the enzymes that catalyzed a series of biochemical reactions from tricarboxylic acid cycle to fatty acid synthesis (as shown in FIG. 12, in which the box denotes differentially expressed genes after the knockout of mLeg1). The expressions of these genes (SCD1/FASN/ACC/ACL) were down-regulated, meaning the attenuation in the fat *de novo* synthesis in the liver of the mLeg1^{Δ/Δ} mice, i.e., the ability to convert other energy substances into fatty acid and store as lipid was greatly attenuated. The reduction in synthesized fat in the liver accounts for the reduction of neutral fat in the vicinity of the blood vessels inside the liver, the reduction of triacylglyceride in the blood of the mLeg1^{Δ/Δ} mice, as well as the reduction of fat deposition in adipose tissues of the mLeg1^{Δ/Δ} mice.

### Embodiment 11

11.1 Detection of the expression level of transcription factors regulating the expression of lipogenesis genes
   There are mainly four transcription factors regulating the expression of lipogenesis genes, i.e., PPARy, *chrebp,* PGC1α and *srebp1c.* Accordingly, the inventors of the present disclosure first studied the expression level of these transcription factors in liver. The experimental method was as follows.
   11.1.1 The expression level of the liver transcription factors (PPARy, *chrebp,* PGC1α and *srebp1c*) in the wild-type mice and the mLeg1^{Δ/Δ} mice was detected using a method similar to that in step 10.1.1, and as to the specific experimental method, reference can be made to steps 2.1-2.4 in Embodiment 2, the related primers used were as shown in Table 1, and the detection results were as shown in FIG. 13.

As could be known from FIG. 13 (in the figure, the ordinate represents the relative expression level, and the abscissa represents the transcription factors regulating lipogenesis), among the four transcription factors (PPARy, *chrebp,* PGC1α and *srebp1c*), only the expression of *srebp1c* was significantly reduced in the liver of the mLeg1^{Δ/Δ} mice. Thus, the reduction of the expression of fatty acid synthetase in the liver of the mLeg1^{Δ/Δ} mice was caused by the reduction of the expression of *srebp1c.*

### Embodiment 12

### 12.1 Akt phosphorylation level in the liver of the mLeg1^{Δ/Δ} mice

There are mainly two ways of regulating the activity of *srebp1c:* one is that since unphosphory-lated *srebp1c* typically resides in cytoplasm, Akt regulates phosphorylation of *srebp1c* through mTORC1, so that *srebp1c* is transferred from the cytoplasm to cell nucleus and exerts its transcriptional activity; and the other is that Akt can positively regulate the transcription level of *srebp1c* using a mechanism that is currently unknown. Thus, the regulation of the activity of *srebp1c* is mainly achieved by the activity of Akt.

In this embodiment, the inventors of the present disclosure detected the activity level of Akt in the lipogenesis center (i.e., liver) and salivary glands in which mLeg1 was expressed. The activity of Akt can be indicated by the phosphorylation level thereof. Hence, the activity of Akt can be reflected by detecting the phosphorylation level of Akt. The experimental method was as follows.

12.1.1 The proteins of liver and salivary glands were extracted by the method in step 3.1, and the phosphorylation level of Akt was detected using the Akt phosphorylation antibody (Cell signaling #4060P) by Western blot.

The detection results were as shown in FIG. 14(a) and FIG. 14(b).

As could be known from FIG. 14(a) and FIG. 14(b) (in which WT represents the wild-type mice, and mLeg1^{Δ/Δ} represents mLeg1 knockout mice), the phosphorylation level of Akt in the liver of the mLeg1^{Δ/Δ} mice was greatly lower than that in the wild-type mice (as shown in FIG. 14(a)), and the difference was especially remarkable for the Akt phosphorylation in the salivary glands, as there was significant phosphorylated Akt protein in the wild-type mice, while substantially no Akt phosphorylation could be detected in the salivary glands of the mLeg1^{Δ/Δ} mice (as shown in FIG. 14(b)). All these results demonstrated that the Akt activity of the mLeg1^{Δ/Δ} mice had been inhibited, which also explained the reduction of *srebp1c* expression.

### Embodiment 13

The factors secreted to the supernatant by salivary gland cells can induce Akt phosphorylation in the HepG2 cells.

In order to verify whether the knockout of mLeg1 was directly related to the attenuation of Akt activity in liver, the inventors of the present disclosure first detected whether mLeg1 could activate Akt by using an *in vitro* experimental system. Since mLeg1 is a secretory protein having enriched expression in the salivary glands of a mouse, if the salivary gland cells are primarily cultured, secreted mLeg1 can be obtained in the cell culture supernatant. Accordingly, the inventors of the present disclosure conducted Western blot detection on the cell culture supernatant of the primarily cultured cells of the salivary glands. The experimental method was as follows.

### 13.1 Primary culture of salivary gland cells:

13.1.1 After the mice were decapitated, and the hair thereof was removed completely. The salivary glands were taken out quickly and washed twice with sterilized PBS. The salivary glands were minced using scissors.
13.1.2 The minced salivary glands were collected in a buffer (with the volume of V) at a ratio of 40 mg/ml. Hyaluronidase, collagenase II and 50 mM CaCl₂ were added into the buffer respectively at 25 µl, 25 µl and 250 µl per 2 ml of buffer for incubation at 37°C for 40 minutes.
13.1.3 The resultant product was centrifuged at 1500 rpm to remove the supernatant, and step 13.1.2 was repeated.
13.1.4 The resultant product was centrifuged at 1500 rpm to remove the supernatant, washed with buffer having a volume of V, centrifuged to remove the supernatant, washed once again with 1/2 V of buffer, and centrifuged to remove the supernatant.
13.1.5 The tissues resulting from centrifugation were re-suspended with 1/2 V of buffer, and filtered using the cell strainer (Cell strainer, BD Cat. No. 352340) to obtain the filtrate which was then cultured using the MSG culture solution.

The formulation of the solution used therein was as follows:
Buffer: (1% BSA (Amresco Cat. No. 0332) in Hank's buffer (Beyotime, Cat. No. C0218)).

The formulation of enzyme: Hyaluronidase (Sangon Biotech, Cat. No. A002594) was dissolved using buffer, and the concentration was 40 mg/ ml; collagenase II (GIBCO Cat. No. 17101-015) was dissolved using buffer, and the concentration was 23mg/ ml. It is preferably that the enzyme solutions are freshly formulated.

MSG culture solution: DMEM high-glucose culture medium (GIBCO Cat. No. 11965-092), IX penicillin and streptomycin (Beyotine, Cat. No. C0222), IX insulin-transferin-Selenium-X (GIBCO, Cat. No. 41400-045), 1µM dexamethasone (Sigma D4902), 10% fetal bovine serum (GIBCO Cat. No. 16000-044).
13.2 Extraction of the total protein of the primarily cultured salivary gland cells: The culture cell suspension obtained in the previous step was centrifuged at 1000 g for 5 minutes to remove the supernatant, added with SDS lysate (63 mMTris-Hcl, pH 6.8, 10% glycerol, 5% β-mercaptoethanol, 3.5% SDS, IX Complete) for lysis, denatured at 100°C for 7 minutes, and was then analyzed by Western blot or stored at -20°C (the adherent cells were treated as follows: after the removal of the culture supernatant, SDS lysate was added thereto, and adherent cells were scraped off with a cell scraper and collected in a 1.5 ml centrifuge tube, denatured at 100°C for 7 minutes, and were then analyzed by Western blot or stored at -20°C).
13.3 The primarily cultured cell culture supernatant was directly collected for the subsequent Western blot detection.
13.4 The method for Western blot detection was the same as that in step 3.2 in step Embodiment 3, and the detection results were as shown in FIG. 14(c).

As could be known from FIG. 14(c) (in FIG. 14(c), CK media represents a cell culture media in which mLeg1^{Δ/Δ} salivary gland cells are cultured, salivary media represents a cell culture solution in which the wild type salivary gland cells are cultured, and salivary cell represents the primarily cultured cells of WT salivary glands), mLeg1 protein was present in both the wild-type cells and the wild-type cell culture supernatant, and by detecting the amount of Akt protein in the cells, it was demonstrated that the mLeg1 in the wild-type cell culture supernatant did not result from cell contamination.

### Embodiment 14

On the basis of the experiment in Embodiment 13, the inventors of the present disclosure cultured human liver cancer cells HepG2 using the mLeg1-containing culture solution (from the wild type salivary gland cell culture supernatant) and mLeg1 protein-free culture solution (from the salivary gland cell culture supernatant of the mLeg1^{Δ/Δ} mice), and studied whether salivary gland secretions could directly promote Akt phosphorylation of liver cancer cells, and whether such ability to induce and activate Akt phosphorylation was the same with and without mLeg1 protein. The experimental method was as follows.
14.1 Culture of human liver cancer cells HepG2: 10% newborn calf serum (GIBCO Cat. No. 16010-159) in DMEM high-glucose culture medium was used to culture the cell line. Cells were kept in a 37°C incubator with 5% CO₂, and saturated humidity. At the time of passage, the culture solution was thoroughly removed, and the left cells was digested with 0.25% trypsin (EDTA-free, Sigma Cat. No. T4549), and an appropriate amount of cells were subjected to subculture or subsequent experiments.
14.2 The adherent HepG2 cells were incubated using the culture supernatant of the primary culture of the wild-type mouse salivary gland cells and the culture supernatant of the mLeg1^{Δ/Δ} mice, and the cell samples were collected 20 minutes later and 10 hours later.
14.3 The content of p-Akt was detected using an Akt phosphorylation antibody by Western blot, so as to reflect Akt phosphorylation level.
14.4 The results were as shown in FIG. 14(d).

As could be known from FIG. 14(d) (in FIG. 14(d), CK represents the salivary gland cell culture supernatant of the mLeg1^{Δ/Δ} mice, and mLeg1 represents the salivary gland cell culture supernatant of the wild-type mice), whether the HepG2 cells were cultured for 10 hours or 20 minutes, the Akt phosphorylation level of the HepG2 cells cultured with the wild type salivary gland cell supernatant was remarkably higher than that of the cells cultured with the mLeg1^{Δ/Δ} supernatant, and the mLeg1 could induce Akt phosphorylation within 20 minutes, demonstrating that the salivary gland secretions of the wild-type mice can indeed promote Akt phosphorylation, and when mLeg1 was knocked out, the ability of the salivary gland secretions to activate Akt was reduced greatly, demonstrating that mLeg1 secreted by the salivary glands can directly or indirectly regulate the activity of Akt.

### Embodiment 15

The factors secreted to the supernatant by the salivary gland cells could induce Akt phosphorylation in the liver of the mLeg1^{Δ/Δ} mice.

The above-described *in vitro* experiments proved that the wild type salivary gland cell secretions can promote Akt phosphorylation of liver cancer cells. Further, the inventors of the present disclosure studied whether these secretions could regulate the phosphorylation level of *in vivo* liver Akt, and the experimental method was as follows.
15.1 The supernatant secreted by the primarily cultured cells of the salivary glands of the wild-type mice and of the mLeg1^{Δ/Δ} mice was injected into the mLeg1^{Δ/Δ} mice respectively by intraperitoneal injection and tail vein injection.
15.2 The mice were sacrificed 1 hour after injection to collect the livers thereof, and whether the Akt phosphorylation level in the liver had been changed was detected using the Akt phosphorylation antibody by Western blot. The results were as shown in FIG. 15(a).

As could be known from FIG. 15 (a) (in FIG. 15(a), WT represents the salivary gland cell culture supernatant of the wild-type mice, mLeg1^{Δ/Δ} represents the salivary gland cell culture supernatant of the mLeg1^{Δ/Δ} mice, lumbar represents intraperitoneal injection, and vein represents tail vein injection), the wild type salivary gland cell secretions, whether through intraperitoneal injection or tail veil injection, could promote liver Akt phosphorylation of the mLeg1^{Δ/Δ} mice. The above results demonstrate that mLeg1-containing salivary gland secretions can also regulate *in vivo* liver Akt phosphorylation. In addition, the results also suggest that the salivary gland secretions can eventually reach liver through transportation.

### Embodiment 16

Induction of Akt phosphorylation by mLeg1 protein of different concentrations partially purified from the salivary glands

The most obvious difference between the salivary gland cells of the wild-type mice and the salivary gland cells of the mLeg1^{Δ/Δ} mice lies in whether they can express mLeg1, then as to whether the difference in Akt phosphorylation caused by the salivary gland cell secretions of the wild-type mice and of the mLeg1^{Δ/Δ} mice directly results from mLeg1, i.e., whether mLeg1 protein can directly induce Akt phosphorylation, the inventors of the present disclosure conducted a study by the following experimental method.
16.1 The level of Akt phosphorylation induced by mLeg1 protein isolated and purified by column chromatography: The experimental method was as follows.
16.1.1 The salivary glands of two to three wild-type mice were harvested and rinsed in the pre-cooled PBS buffer, minced into fine pieces with scissors, and then transferred to a tissue homogenizer, added with 4 ml of lysate buffer (50 mM Tris-Hcl, pH 8.0, 150 mM NaCl, 0.5% NP40, 2x complete), fully homogenized on ice by pipetting through a 23G needle repeatedly, incubated for 30 minutes at 4°C in a vertical shaker, and then centrifuged at 12000 g at 4°C for 15 minutes to collect the supernatant.
16.1.2 The supernatant was passed through a sepharose 6B gel filtration column at a constant speed at 4°C, wherein the PBS buffer was used for elution, and different eluted components were separately collected by 4 ml per tube.
16.1.3 The content of mLeg1 in each tube was detected by Western blot. The tube with the highest mLeg1 content was taken as partially purified mLeg1, and the concentration of mLeg1 protein was estimated using the recombinantly-expressed mLeg1 protein in *Escherichia coli* as a reference, for subsequent experiments.
16.1.4 The partially purified mLeg1 protein was diluted to different concentrations (3.14x10⁻²ng/ µl, 0.314ng/ µl and 3.14ng/ µl), and the different concentrations of mLeg1 protein were added to HepG2 culture medium respectively to culture HepG2, followed by incubation at 37°C for 20 minutes, then total protein was extracted (as to the method of extraction, reference can be made to step 13.2), and Akt phosphorylation level at the different concentrations was detected by Western blot (referring to step 14.3, anti-p-Akt antibody (S473, Cell signaling #4060P) was selected as the corresponding antibody, and the dilution ratio was 1:1000). The results were as shown in FIG. 15(b).

As could be seen from FIG. 15(b) (in FIG. 15(b), A represents 3.14ng/ µl, B represents 0.314ng/ µl, C represents 3.14x10⁻²ng/ µl, and "-" represents no addition), mLeg1 protein could induce Akt phosphorylation, and partially purified mLeg1 at nanogram levels would be able to induce Akt phosphorylation of the HepG2 cells.
16.2 The level of Akt phosphorylation induced by the components from the salivary glands of the wild-type mice and of the mLeg1^{Δ/Δ} mice: The experimental method was as follows.
16.2.1 mLeg1 protein-enriched components from wild-type mice and from mLeg1^{Δ/Δ} mice were extracted respectively.
16.2.2 The components extracted in step 16.2.1 were respectively added to the HepG2 culture medium to culture HepG2, followed by incubation at 37°C for 20 minutes, then the total protein of the cultured cells was extracted therefrom (as to the method of extraction, reference can be made to step 13.2)
16.2.3 The Akt phosphorylation level at different concentrations was detected by Western blot (reference can be made to step 13.2 for details, anti-p-Akt antibody (S473, Cell signaling #4060P) was selected as the corresponding antibody, and the dilution ratio in use was 1:1000). The results were as shown in FIG. 16(a).

As could be seen from FIG. 16(a) (in FIG. 16(a), "+" represents mLeg1-enriched component of the wild-type mice incubation, and "-" represents the corresponding component from mLeg1^{Δ/Δ} mice incubation), the ability of the mLeg1-containing components in the salivary glands of the wild-type mice to induce Akt phosphorylation was significantly higher than that of the corresponding components in the salivary glands of the mLeg1^{Δ/Δ} mice.

### Embodiment 17

Activation of Akt by mLeg1 is dependent on PI3K pathway

mLeg1 is a secretory protein, and in the experiment of incubating HepG2 cells with partially purified mLeg1, mLeg1 is equivalent to an extracellular protein. Moreover, the phosphorylation of Akt is an important intracellular signal transduction process. Thus, the process of converting an extracellular signal to activate an intracellular signal is involved herein. Throughout the known extracellular signal-induced Akt phosphorylation, it relies primarily on the mechanism that PI3K phosphorylates PIP2 and converts it to PIP3, thereby further inducing phosphorylation of Akt. Therefore, the mLeg1 induced Akt phosphorylation may also rely on PI3K pathway. The inventors of the present disclosure used PI3K specific inhibitor LY2940002 to inhibit PI3K signaling pathway, and observed whether the ability of mLeg1 to activate Akt was changed after the PI3K pathway was inhibited. The experimental method was as follows.
17.1 Before mLeg1 treatment, the HepG2 cells were cultured overnight in a starved state using 0.1% serum, and digested with 0.25% trypsin (EDTA-free, Sigma Cat. No. T4549), and then a proper amount of cells were taken therefrom and placed in a centrifuge tube.
17.2 The supernatant harvested from primary culture of the salivary gland cells of the wild type (containing mLeg1) mice and of the mLeg1^{Δ/Δ} (not containing mLeg1) mice was used to culture the HepG2 cells, and PI3K inhibitors LY294002 (cell signaling) at different concentrations (10µm, 20µm and 40µm in a concentration reducing sequence) were added to the culture media to inhibit PI3K pathway.
17.3 The Akt phosphorylation level at different concentrations of LY294002 was detected by Western blot. As to the specific operations, reference can be made to step 18.2.2. The detection results were as shown in FIG. 16(b).
17.4 In addition, after step 17.1, the HepG2 cells were cultured using mLeg1 partially purified by column chromatography and 10µM LY294002 in a cell incubator for 15 minutes, followed by centrifugation at 1000 g for 5 minutes to remove the supernatant. SDS lysate was then added thereto to lyse the cells so as to extract protein (as to the specific operations, reference may be made to step 13.2).
17.5 The Akt phosphorylation level of the HepG2 cells was detected by Western blot. The detection results were as shown in FIG. 16(c).

As could be seen from FIG. 16(c) (in FIG. 16(c), WT media represents the culture supernatant of the primarily cultured salivary gland cells of the wild-type mice, "-" represents no LY294002 addition, A represents the addition concentration of 10µM, B represents the addition concentration of 20 µM, C represents the addition concentration of 40 µM, and CK media represents the culture supernatant of the salivary gland cells of the mLeg1^{Δ/Δ} mice), the ability of the culture supernatant from WT salivary cell (WT media) to activate Akt was significantly higher than that from mLeg1^{Δ/Δ} mice cell (CK media), and Akt phosphorylation induced by the WT media could be remarkably inhibited by the addition of the PI3K inhibitor LY294002 at different concentrations to WT media. This indicates that when LY294002 was added, the ability of mLeg1-containing culture solution to induce Akt phosphorylation was greatly compromised, and intracellular Akt phosphorylation was kept at a very low level. The PTEN phosphorylation level did not increase with the addition of LY290004, demonstrating that such inhibition of the Akt phosphorylation level was not caused by PTEN.

Furthermore, as could be known from FIG. 16(c) (in FIG. 16(c), "-" in the upper row represents no addition of mLeg1, "+" in the upper row represents addition of mLeg1; "-" in the lower row represents no addition of LY294002, and "+" in the lower row represents addition of LY294002), by adding the column chromatography purified mLeg1 and 10 µM LY290004 into the HepG2 cell culture media the inventors of the present disclosure found that LY294002 was able to completely inhibit the induction of Akt phosphorylation by mLeg1. Thus, the mLeg1 induced Akt phosphorylation relies on the PI3K signaling pathway.

### Embodiment 18

### Activation of Akt by mLeg1 through RTK

Transduction of extracellular signal into a cell needs a bridge in the cell membrane. One kind of the membrane proteins connecting an extracellular signal and intracellular PI3K signal is receptor tyrosine kinase (Receptor tyrosine kinase, RTK). After bound with a corresponding ligand, RTK itself can be phosphorylated and also phosphorylate the downstream substrates, and such phosphorylation occurs at tyrosine residues. Therefore, it is possible to determine whether the mLeg1 induced Akt phosphorylation is realized by RTK by detecting the difference in the tyrosine phosphorylation level. The experimental method was as follows.
18.1 Partially purified mLeg1 was added to the HepG2 cell medium to culture the HepG2 cells, and then total protein was extracted.
18.2 The phosphorylation level of the total intracellular tyrosine was detected through the tyrosine phosphorylation antibody 4G10 (Millipore, 05-321). The results were as shown in FIG. 16(d).

As could be known from FIG. 16(d) (in FIG. 16(d), CK represents no addition of mLeg1), after the addition of mLeg1 protein, the phosphorylation level of the intracellular tyrosine was far higher than that of the control group. In addition, the Western blot detection results also indicate that the proteins undergoing tyrosine phosphorylation all have a relatively large molecular weight, and this tallies with the fact that RTKs all have a relatively large molecular weight, further suggesting that it is very likely that mLeg1 transduces its signal through RTK.

### Embodiment 19

### Activation of EGFR by mLeg1 protein

In human, there are 58 different RTKs in total. Accordingly, the inventors of the present disclosure decided to study which RTK is responsible for mLeg1 signal transduction. Here the inventors of the present disclosure selected the RTK screening system of R&D. In this system, 49 RTK antibodies are crosslinked on a single membrane, and by these antibodies, the corresponding RTK proteins are pulled down from the cell lysate and attached onto the membrane. On the basis of the property that activation of RTK will result in phosphorylation of tyrosine in the RTK itself, it is feasible to detect the phosphorylation level of the attached RTK tyrosine through the tyrosine phosphorylation antibodies, so as to indicate the RTK activation conditions. The experimental method was as follows.

19.1 The screening of receptor tyrosine kinase was performed by a RTK assay kit (Proteome Profiler Human Phospho-RTK Array Kit, R&D Cat. no. ARY001B) according to the manufacturer's instruction. The kit can detect 49 of 58 RTKs in total. The operation steps are summarized as follows: mLeg1 and a control were used to treat the cells (cells were grown in a 10 cm petri dish) separately, and then 500 µl of lysis buffer17 was used to lyse the cells; the RTK screening membrane was blocked for 1 hour using Assay buffer1 and then coated with a cell lysate for binding overnight, the resultant product was washed three times with IX Wash Buffer, 10 minutes each time, then added with Anti-Phospho-Tyrosine-HRP Detection Antibody diluted with IX Array Buffer2 at the ratio of 1:5000 for incubation at room temperature for 2 hours; followed by washing 3 times with IX Wash Buffer, 10 minutes each time, then applied with Chemi Reagent Mix and developed, and finally imaged in a fluorescent chemiluminescent imager (Clinx Science Instruments Cat. No. 3400) with the signals recorded. The results were as shown in FIG. 17.

As could be known from FIG. 17, after the addition of mLeg1, the activity of most of the RTKs in the HepG2 cells was not influenced and was kept at a relatively low level, and only, tyrosine phosphorylation in EGFR increases significantly (as indicated by the circled points in FIG. 17), meaning that the addition of mLeg1 activated the EGFR, and further activated downstream Akt signal.

Further, the inventors of the present disclosure detected the impact of mLeg1 on intracellular EGFR activation level by an EGFR phosphorylation specific antibody. The results of FIG. 18(a) (in FIG. 18(a), "-" represents no addition, and "+" represents mLeg1 addition) also showed that the addition of mLeg1 could activate the EGFR very rapidly, and activate Akt signals thereafter.

### Embodiment 20

The activation of Akt by mLeg1 is dependent on the activation of EGFR

The RTK screening results in Embodiment 19 show that mLeg1 very likely induced Akt phosphorylation through EGFR activation. If the inhibition of EGFR activity by an EGFR inhibitor can block mLeg1-induced Akt phosphorylation, it can be further demonstrated that mLeg1 activates Akt through EGFR. Here, the inventors of the present disclosure selected the EGFR specific inhibitor AG1478 to inhibit the activity of EGFR. The experimental method was as follows.
20.1 Culture of the HepG2 cell: Reference may be made to step 18.1 for details.
20.2 Additives (BSA, mLeg1, AG1478) were added to the cell culture solution separately, after culture for 15 minutes, the total protein was extracted (referring to step 13.2), and the level of the proteins (p-Akt, Akt, P-EGFR) in each treatment group was detected by Western blot (referring to step 2.2). The results were as shown in FIG. 18(b).

As could be known from FIG. 18(b), when column chromatography partially purified mLeg1 was added to the culture medium of the HepG2 cells, the Akt phosphorylation could be induced, while in the BSA control group to which bovine serum albumin was added, Akt phosphorylation was substantially not influenced. When 1µM AG1478 was further added to the culture medium to inhibit EGFR activity, the inventors of the present disclosure discovered that the mLeg1-induced Akt phosphorylation was blocked. Moreover, by the study on the EGFR phosphorylation level, it was found that the treatment of mLeg1 induced EGFR phosphorylation, but after the addition of AG1478, activation of EGFR was inhibited. Thus, mLeg1's induction of Akt phosphorylation relies on the activation of the cell membrane receptor, EGFR.

### Embodiment 21

### Existence of protein-protein interaction between mLeg1 and EGFRs

The above results show that mLeg1 can activate PI3K through EGFR, thereby inducing Akt phosphorylation. EGFR is a receptor protein on the surface of a cell membrane, and mLeg1 is a secretory protein. Thus, mLeg1 may be directly bound with EGFR to serve as a signaling molecule so as to activate downstream signals. Therefore, the inventors of the present disclosure then detected whether there was interaction between mLeg1 and EGFR by co-immunoprecipitation.

Since the above results show that mLeg1 can influence the functions of liver, the inventors of the present disclosure used the column chromatography partially purified mLeg1 protein to incubate the cells isolated from liver homogenate. Since the reaction of mLeg1 activating EGFR is very short and the EGFR will be degraded soon after being activated, the inventors of the present disclosure used mLeg1 to incubate the liver cells at 4°C, and simultaneously added a crosslinking agent DSP thereto to stabilize the interaction between mLeg1 and its potential interacting protein, and then washed mLeg1 and lysed the liver cells using NP40 lysate for co-immunoprecipitation. The experimental method was as follows.
21.1 Antibody crosslinking: 30 µl of protein A/G (beyotime Cat. No. P2012) was centrifuged at a low speed (500-1000 g) to remove the supernatant, and then washed twice using 4°C pre-chilled PBS, then added with mLeg1 antibody (20 µg of antibody were dissolved in IX 100 µl PBS) for incubation at room temperature for 30 minutes, and then centrifuged to remove the supernatant. The beads were washed three times with 300 µl of PBS, then incubated with 50 µl of DSS solution (5 µl 10x PBS, 36 µl H₂O, 9 µl 2.5 mM DSS (Thermo, Cat. NO. 21655)) for 50 minutes at room temperature. After centrifugation to remove supernatant, the beads were washed three times with 50 µl of 100 mM pH 2.2 glycine, washed twice with 300 µl of PBS containing 1% NP40, and finally washed once with 300 µl of PBS. The beads crosslinking antibodies were kept wet, and were centrifuged before use to remove the supernatant completely.
21.2 By column chromatography purification, mLeg1-containing components was obtained from wild-type mice salivary glands and corresponding components was obtained from mLeg1^{Δ/Δ} mice salivary glands.
21.3 The cells isolated from liver homogenate were incubated using the above components at 4°C, respectively, and at the same time, the crosslinking agent DSP was added to stabilize the interaction between mLeg1 and its potential interacting protein.
21.4 Co-immunoprecipitation: The tissues or cells were homogenized sufficiently by NP40 lysate (50 mMTris-Hcl, pH 8.0, 150 mM NaCl, 1% NP40, 2Mm EDTA, 1 mM PMSF, 2x complete), and lysed for 15 minutes at 4°C in a vertical shaker, and then centrifuged by 12000 g at 4°C for 15 min to collect the supernatant for subsequent operations. The antibody crosslinked beads were added to the supernatant for incubation at 4°C overnight; the beads were spun down and washed three times with 4°C pre-chilled PBST (0.1% Tween 20) and then washed twice with pre-chilled PBS; 50 µl of 100 mM pH 2.2 glycine was added to elute the protein bound with the beads, 2 µl of 1M pH9.5 glycine was added to neutralize pH, and the resultant product was stored at -20°C or used directly for subsequent analysis.
21.5 The protein pulled down by the mLeg1 antibody was detected by Western blot. The results were as shown in FIG. 18(c).

As could be known from FIG. 18(c) (in FIG. 18(c), input represents 1/50 of the protein used for co-immunoprecipitation experiment; "-" represents no mLeg1 added into the liver homogenate; "+" mLeg1 added into the liver homogenate; and IP:α-mLeg1 represents using mLeg1 antibody to pull down mLeg1 and its interacting protein. Without mLeg1, EGFR in the liver could not be pulled down by the mLeg1 antibody, while together withmLeg1, EGFR could be co-precipitated by mLeg1 antibody, therefore, there was indeed interaction between mLeg1 and EGFR.

### Embodiment 22

The above study indicates that the function of mLeg1 in regulating the liver Akt activity is realized by binding to and activating EGFR, but this requires that mLeg1 can reach the liver to bind to EGFR. The inventors of the present disclosure know that mLeg1 is expressed in the salivary glands and secreted in saliva, but it remains a question as to whether such mLeg1 in the oral cavity can reach the liver and interact with EGFR in the liver. In order to simulate this condition, study was conducted by the following experimental method.
22.1 The column chromatography partially purified mLeg1 protein was intragastrically administered to the mLeg1^{Δ/Δ} mice at 50 ng of mLeg1 per gram of body weight of the mice.
22.2 The mice were sacrificed at different time points (0, 10, 20, 40, 60 min) after intragastric administration and liver samples were collected, an immunoprecipitation experiment was conducted to pull down mLeg1 protein (if there is any) and its interacting protein in the liver by using mLeg1 antibodies Reference may be made to steps 21.2-21.5.

The behavior of the mLeg1 secreted by the salivary glands after entering the digestive tract was simulated by means of the method above.

Based on the abovementioned theory, if salivary expressed mLeg1 can be transferred to liver, then there should be mLeg1 protein detected in the liver after intragastric administration. The results were as shown in FIG. 18(d).

As could be known from FIG. 18(d), mLeg1 protein could be detected in the liver of the mice just 10 minutes after intragastric administration, the amount of the protein reached the maximum value at 20 minutes after intragastric administration, and mLeg1 in the liver decreased at 40 minutes and 60 minutes after intragastric administration. Moreover, the inventors of the present disclosure observed that the amount of EGFR that interacts with mLeg1 reached the maximum 10 minutes after mLeg1 intragastric administration, and then the amount was continuously reduced as time lapsed. This may result from the fact that mLeg1 binds with EGFR rapidly, and transmitted downstream signals rapidly, as in *in vitro* experiments, mLeg1 can activate EGFR within 1 minute and the phosphorylation level of EGFR began to decrease within 3 minutes. Further, the inventors of the present disclosure also detected the activation of such intragastrically administered mLeg1 protein on downstream Akt. Akt phosphorylation in the liver increased 10 minutes after intragastric administration of mLeg1, and this increase was more significant 40 minutes to 60 minutes after intragastric administration. According to the above results, mLeg1 secreted from the salivary glands can be transferred into the liver and activates EGFR in the liver, thereby activating Akt to finally regulate the physiological functions of the liver cells.

### Embodiment 23

Competitive inhibiting of the functions of wild type mLeg1 by modification-defective mLeg1 protein

*In vivo* proteins often need to be properly modified in order to function properly. Thus, modification-defective mLeg1 protein may lose its ability to activate Akt, and may also competitively inhibit the functions of the wild type mLeg1. In order to test this hypothesis, the inventors of the present disclosure used an *Escherichia coli* expression system to express recombinant mLeg1 protein (mLeg-Re). Since *Escherichia coli* is prokaryote, eukaryotic proteins expressed in this expression system tend to lose proper modification. The experimental method was as follows.

23.1 PCR amplification was conducted using salivary gland cDNA as a template and using the primers mLeg1 ATG BamHIfw: CTCAGTggatccATGGCTGTCCTGGCTTCC and mLeg1 TAA XhoIRv: TACCTCGAGAGAAGATGTTGCCAGGAACTCTT. The reaction conditions were: 95°C for 3 minutes, 95°C for 30 seconds, 58°C for 30 seconds, 72°C for 1 minute, and after 34 cycles, 72°C for 10 minutes. The PCR product was purified, and was then subjected to BamHI and XhoI digestion for overnight. Meanwhile, the Pet30(a) vector was digested with the same restriction enzyme. After being purified, 50 ng of PCR product after restriction enzyme digestion and 50 ng of vector after restriction enzyme digestion were linked using T4 DNA ligase. After reaction for 10 minutes at room temperature, the linked product was transformed using competent cell DH5α. The steps were as follows: the mixture of the ligated product and DH5α cells was placed on ice for 30 minutes, heat-shocked at 42°Cfor 90 seconds, placed on ice for 2 minutes, then added with 1 ml of LB culture medium, and shaken at 37°C for 1 hour. 100 µl of bacteria solution was applied to KANA-resistant LB plate overnight. Monoclone was selected, and correct insertion of the mLeg1 fragment in the monoclone was determined by sequencing. The correct single colony was subject to expand culture overnight using KANA-resistant LB culture medium, and then plasmids were extracted therefrom.

The process of expressing the mLegl-Re protein was as follows: The extracted plasmids were transformed into DE3 expression competent cells, and also applied to a plate. The single colony was selected and sequenced to verify the presence of correct mLeg1 plasmids in the colony. The single colony was cultured overnight with KANA-resistant LB. 10 ml of the overnight cultured bacteria solution was diluted in 1L of KANA-resistant LB, shaking-cultured at 37°C to reach a bacteria solution OD value of 0.5, and then added with 1 mM IPTG to induce the expression of mLegl-Re protein. Continue culturing the bacteria for 4 hours more, and centrifuged to collect the bacterial pellet. 10 ml of lysis buffer (50 mM NaH₂PO₄, 300 mM NaCl and 10 mM imidazole, pH 8.0) was added to resuspend the bacteria pellet, and 1mg/ ml lysozyme was added thereto for reaction on ice for 1 hour. The bacterial solution was broken up by sonication and the supernatant was collected after centrifugation. Then 4 ml of Ni-NTA (QIAGEN, 30230) beads were added thereto the supernatant to bind with the mLeg1 recombinant protein overnight. The beads were centrifuged at 4°C at 1000 g to remove the supernatant, washed twice with 4 ml of washing solution buffer (50 mM NaH₂PO₄, 300 mM NaCl and 20 mM imidazole, pH 8.0), and then was eluted with 4 ml of eluent buffer (50 mM NaH₂PO₄, 300 mM NaCl and 250 mM imidazole, pH 8.0). The eluted product was placed in a semipermeable membrane (Thermo, SnakeSkin Dialysis Tubing, #68100), dialyzed with PBS for 3 days, with PBS replaced every 12 hours. The finally obtained protein was mLegl-Re protein, which was stored at -80°C after the concentration was measured.
23.2 The size of the mLegl-Re protein was detected by Western blot. The results were as shown in FIG. 19(a).
23.3 The extracted recombinant protein mLegl-Re and the wild type mLeg1 protein were used to incubate the HepG2 cells, and then the Akt phosphorylation level of the HepG2 cell was detected. As to the details, reference can be made to step 18.2.2. The results were as shown in FIG. 19(b).

The immunoblotting results show that the size of the mLegl-Re protein was greatly smaller than that of the wild-type mLeg1 protein (as shown in FIG. 19(a)). When the HepG2 cells were incubated using the recombinant protein mLegl-Re alone, the mLegl-Re protein indeed lost the ability to induce Akt phosphorylation. The partially purified wild-type mLeg1 protein (50 ng) was mixed with excess mLegl-Re protein (500 ng) for 10 minutes, and then used to incubate HepG2 cell for 10 minutes, and then the Akt phosphorylation level was detected in these cells. The results showed that under this situation the mLeg1 protein was still able to induce Akt phosphorylation. This means that the mLegl-Re protein cannot inhibit the functions of the mLeg1 protein in this case. But when the HepG2 cells were incubated with mLegl-Re protein for 10 minutes firstly, followed by addition of the mLeg1 protein, the results showed that the ability of the mLeg1 protein to activate Akt was completely inhibited (as shown in FIG. 19(b)). It is highly likely attributed to the fact that the mLegl-Re protein is also capable of competitively binding to the EGFR, thereby blocking the ability of the wild-type mLeg1 to bind to the EGFR and activate downstream Akt.

Further, as to whether the mLegl-Re protein can also competitively inhibit the functions of the wild-type mLeg1 in mice, thereby inhibiting the lipogenesis pathway and finally blocking the occurrence of obesity, it was verified by the following experimental method.

23.4 10-weeks old wild type mice having the similar body weight (t1) were randomly divided into 3 groups with 3 to 4 mice in each group. The wild type mice were fed with high-fat diet and intragastric administration with 20 µg of mLegl-Re protein every day, which served as mLegl-Re administration experimental group. Another group of wild type mice of the same age were fed with the same high-fat diet and intragastric administration with the same amount of bovine serum albumin (BSA) every day, which served as BSA administration control group. The left group of wild type mice at the same age were fed with the same standard chow diet, which served as another control group. One month later, the body weight (t2) of these mice were measured, and the results were represented by increased body weight (t2-t1). The experiment was repeated twice, and the final body weight changes (i.e., increased body weight) were averaged. The results were as shown in FIG. 20(a).

As illustrated by FIG. 20(a), due to high-fat-diet feeding, the BSA control group exhibited a significant body weight increase, as compared with the mice fed with standard chow diet, while the mice in the mLegl-Re protein administration experimental group had a body weight increase almost the same as that of the mice fed with standard chow diet, and they are both significantly lower than the BSA control group. This suggests that the mLegl-Re protein has the function of inhibiting obesity or body weight increase caused by over-eating high-fat diet.

These results further imply that the mLegl-Re protein can bind to the EGFR in liver and block the normal functions of mLeg1. In order to further verify this hypothesis, the inventors administered the mLegl-Re protein to the mLeg1^{Δ/Δ} mice by intragastric administration, and detected, by the method of co-immunoprecipitation, whether the mLegl-Re protein could reach the liver and bind to EGFR in the liver. The results were as shown in FIG. 20(b) (in the figure, input represents the protein used for co-immunoprecipitation experiment, "-" represents the control group without mLegl-Re administration; and "+" represents the experimental group with mLegl-Re administration; IP:α-mLeg1 represents using mLeg1 antibody to pull down the mLeg1 protein and the interacting protein thereof): two hours after the intragastric administration, mLegl-Re protein could be detected in the liver of the mLeg1^{Δ/Δ} mice, and significant EGFR protein level could be detected in the protein pulled out down together with mLeg1 by the mLeg1 antibody. The results demonstrate that mLegl-Re protein can indeed reach the liver from digestive duct, and bind to EGFR to block the normal functions of mLeg1, and finally reduce the lipogenesis.

In summary, when normal mice are continuously fed with high fat diet (10%), the body weight of the mice will continuously increase, finally resulting in obesity and a series of obesity syndromes. When the mLeg1 gene is knocked out, i.e., the functions of mLeg1 are inhibited, even fed continuously with high fat diet, the mice have a body weight increase that does not significantly differ from that of the mice fed with standard chow diet, and do not develop obesity symptoms. This means that the inhibition of the functions of mLeg1 can inhibit overeating-induced obesity. mLeg1 fulfils its function of regulating lipogenesis by an EGFR-Akt-Srebplc signal axis, suggesting that any compound capable of interfering with the functions of any of the factors mLeg1, EGFR, Akt and Srebplc may be potential to be developed into a drug inhibiting diet-induced obesity. Therefore, inhibiting the functions of the mLeg1-EGFR-Akt-Srebp1c signal axis and blocking the de novo lipogenesis have the potential to become novel approaches for the treatment of obesity. The knockout of Legl homologous genes or downregulation of the expression or inhibit activity of Legl by a particular compound in agricultural animals can reduce fat accumulation.

In addition, mLeg1 protein can promote lipogenesis by the EGFR-Akt-Srebplc signal axis, thus mLeg1 can be used for the preparation of a drug for promoting lipogenesis, which, on the one hand, can be used for the treatment of lipopenia, including lipopenia caused by chemotherapy, and on the other hand, can be used for weight gaining, including weight gaining of thin and weak people, fattening of domestic animals, etc.

Furthermore, the mLeg1 protein can activate Akt signals by interacting with EGFR. Moreover, previous studies indicate that an important mechanism of the development of diabetes is that the liver of the diabetics is resistant to insulin signals, so that insulin cannot activate Akt signals well, and therefore the GLUT2 protein in cytoplasm of liver cells cannot be transported to the surface of cell membranes, making it impossible for blood glucose to be transported into liver to be converted, and finally resulting in a too high blood glucose content. The mLeg1 protein can activate Akt independent of insulin pathway, meaning that the mLeg1 protein is a potential drug for treating diabetes.

Since the mLeg1 (Legl) protein is conserved in all vertebrates, the Legl proteins in these species (including human hLeg1 protein) have similar functions. Thus, the Legl proteins in these species and the functions and uses concerning the Legl-EGFR-Akt-Srebplc signal axis all fall within the protection scope of the present disclosure.

To sum up the abovementioned study results, the main conclusions of the present disclosure can be drawn as follows:
(1) The lipid content in the mLeg1^{Δ/Δ} mice is reduced, and the mLeg1^{Δ/Δ} mice are resistant to obesity caused by high fat diet feeding.
(2) The attenuation in Akt activity in the liver of the mLeg1^{Δ/Δ} mice leads to the attenuation in lipogenesis ability.
(3) mLeg1 can promote Akt phosphorylation in human liver cancer HepG2 cells and the liver of mice.
(4) mLeg1 activates EGFR by interacting with EGFR, and further activates Akt through the EGFR/PI3K signal axis. When inhibiting either EGFR or PI3K signaling, mLeg1 can no longer activate Akt.
(5) In the mice, exogenous mLeg1 in liver is involved in the regulation of the activity level of liver Akt. mLeg1 expressed by salivary gland is secreted into saliva, and enters the digestive tract, and finally reaches the liver to regulate liver lipogenesis. The mLeg1 protein entering the digestive tract by intragastric administration can reach the liver within 10 minutes, and bind to EGFR and activate downstream Akt signals.

Detailed study has been conducted on mLeg1 of which the relevant functions have never been reported. The study indicates that the mLeg1 protein is a new regulatory factor for lipid metabolism. The mLeg1 protein is encoded by mLeg1 gene and has enriched expression in salivary glands, the mLeg1 protein produced in salivary glands is secreted into saliva, and can be transported to liver, activate the PI3K-Akt-Srebplc signaling pathway by binding to the hepatocyte surface receptor EGFR, and regulate the *de novo* synthesis pathway of fatty acid in the liver, so as to regulate the entire lipid metabolism of organisms.

The study indicates that the mLeg1 protein is mainly expressed in salivary glands, thus, the mLeg1 protein can serve as a molecular marker for the morphology of salivary glands and diagnosis of diseases.

The study further indicates that mLeg1 is mainly expressed by salivary glands, thus, the mLeg1 promoter can be used for the preparation of transgenic animals in which certain gene is expressed only in salivary glands, including the preparation of mice in which salivary glands Cre is specifically expressed, etc.

In summary, in the present disclosure, very comprehensive study has been conducted on the functions of mLeg1 gene (on which no study has been conducted in the prior art) by approaches in Genetics, Molecular Biology, Biochemistry and Cell Biology, with the mLeg1 knockout mice as the subject. The study results show that the mLeg1 protein can regulate *in vivo* Akt signals through EGFR, and further regulate *in vivo* lipogenesis. The results show that the mLeg1 gene and protein are closely related to *in vivo* lipogenesis, and further show that the Legl gene or Legl protein in vertebrates including human can be used as a target gene or a target protein for the preparation of drugs related to lipogenesis. The study results provide a brand-new drug target and a new therapeutic approach and idea for the development of drugs in the fields of treatment or prevention of human obesity later in the future, physical recovery or weight gaining of cancer patients after chemotherapy, treatment of diabetes and so forth, and the development in the field of drugs related to fat accumulation in vertebrates.

The above descriptions are only preferred embodiments of the present disclosure, which are not used to restrict the present disclosure. For those skilled in the art, the present disclosure may have various changes and variations. Any modifications, equivalent substitutions, improvements etc. within the spirit and principle of the present disclosure shall all be covered by the protection scope of the present disclosure.

## Claims

1. A Legl protein, **characterized by** having an amino acid sequence as set forth in (1), (2), (3) or (4):
(1) SEQ ID NO: 1;
(2) SEQ ID NO: 2;
(3) a derivative sequence that is obtained by subjecting a sequence as set forth in SEQ ID NO: 2 to substitution and/or deletion and/or addition of a plurality of amino acid residues and has a same or antagonizing bioactivity as SEQ ID NO: 2;
(4) a derivative sequence that is obtained by subjecting the sequence as set forth in SEQ ID NO: 2 to substitution, deletion or addition of one amino acid residue and has a same bioactivity or antagonizing as SEQ ID NO: 2.

2. A use of the Legl protein according to claim 1 as a target protein in preparation or screening of a drug for treating obesity or reducing weight, **characterized in that** the drug is a drug inhibiting a level of the Legl protein; or the drug is a drug blocking binding of the Legl protein to an epidermal growth factor receptor (EGFR) protein; or the drug is a drug inhibiting activity of the Legl protein.

3. A use of the Legl protein according to claim 1 as a target protein in a preparation or screening of a drug for treating lipopenia or gaining weight, **characterized in that** the drug is a drug enhancing a level of the Legl protein; or the drug is a drug promoting binding of the Legl protein to the epidermal growth factor receptor (EGFR) protein; or the drug is a drug enhancing activity of the Legl protein.

4. A use of the Legl protein according to claim 1 for treating lipopenia or gaining weight.

5. A use of the Legl protein according to claim 1 as a target protein in preparation or screening of a drug for treating human diabetes, **characterized in that** the drug is a drug activating an Akt signaling pathway by enhancing a level of the Legl protein or enhancing activity of the Legl protein, so as to stimulate the transportation of GLUT2 to a surface of a cell membrane.

6. A Legl gene, **characterized by** encoding the Legl protein according to claim 1.

7. A use of the Legl gene according to claim 6 as a target gene in preparation or screening of a drug for treating obesity or reducing weight, **characterized in that** the drug is a drug inhibiting an expression level of the Legl gene.

8. A use of the Legl gene according to claim 6 as a target gene in preparation or screening of a drug for treating lipopenia or gaining weight, **characterized in that** the drug is a drug enhancing an expression level of the Legl gene.

9. A use of the Legl gene according to claim 6 as a target gene in preparation or screening of a drug for treating human diabetes, **characterized in that** the drug is a drug activating an Akt signaling pathway by enhancing an expression level of the Legl gene, so as to stimulate the transportation of GLUT2 to a surface of a cell membrane.

10. A use of an RNA interference vector of the Legl gene according to claim 6 in preparation of a drug for treating obesity or reducing weight, **characterized in that** the RNA interference vector silences an expression of the Legl gene.

11. A use of the Legl gene according to claim 6 as a target gene in preparation of a drug for regulating fat accumulation in vertebrates, **characterized in that** the drug is a drug enhancing an expression level of the Legl gene; or the drug is a drug reducing or silencing the expression level of the Legl gene.

12. A use of the Legl gene according to claim 6 in breeding a high-fat-content vertebrate strain, **characterized by** comprising: introducing a plasmid vector linked with the Legl gene into a target animal cell, differentiating and culturing the same to produce a complete vertebrate, the Legl gene being driven by a strong promoter to express.

13. A use of the Legl gene according to claim 6 in breeding a low-fat-content vertebrate strain except human, **characterized by** comprising: knocking out the Legl gene from the vertebrate strain.

14. A recombinant Legl protein, **characterized by** being obtained by subjecting the Legl gene according to claim 6 to recombinant expression and purification in a prokaryotic expression system.

15. A use of the recombinant Legl protein according to claim 14 for treating obesity or reducing weight.

16. A modified Legl protein, **characterized by** being obtained by modifying one or more amino acid residues in the Legl protein according to claim 1, the modification being one or more of glycosylation modification, acetylation modification, methylation modification and phosphorylation modification.

17. A use of the modified Legl protein according to claim 16 for treating obesity or reducing weight, treating human diabetes, treating lipopenia, or promoting fat accumulation in human body.

18. A drug for treating obesity or reducing weight, **characterized in that** the drug is a drug inhibiting a level of the Legl protein, with the Legl protein according to claim 1 as a target; or
the drug is a drug blocking binding of the Legl protein to an epidermal growth factor receptor (EGFR) protein, with the Legl protein according to claim 1 as a target; or
the drug is a drug inhibiting activity of the Legl protein, with the Legl protein according to claim 1 as a target; or
the drug is a drug inhibiting, with the Legl gene according to claim 6 as a target, an expression level of the Legl gene; or
the drug is an RNA interference vector for silencing expression of the Legl gene.

19. A drug for treating lipopenia or gaining weight, **characterized in that** an active ingredient of the drug is the Legl protein according to claim 1; or
the drug is a drug enhancing a level of the Legl protein, with the Legl protein according to claim 1 as a target; or
the drug is a drug promoting binding of the Legl protein to an epidermal growth factor receptor (EGFR) protein, with the Legl protein according to claim 1 as a target; or
the drug is a drug enhancing activity of the Legl protein, with the Legl protein according to claim 1 as a target; or
the drug is a drug enhancing, with the Legl gene according to claim 6 as a target, an expression level of the Legl gene.

20. A drug for treating human diabetes, **characterized in that** an active ingredient of the drug is the Legl protein according to claim 1; or
the drug is a drug activating an Akt signal by enhancing a level of the Legl protein or enhancing activity of the Legl protein, with the Legl protein according to claim 1 as a target, so as to stimulate the transportation of GLUT2 to a surface of a cell membrane; or
the drug is a drug activating an Akt signaling pathway by enhancing an expression level of the Legl gene according to claim 6, so as to stimulate the transportation of GLUT2 to a surface of a cell membrane.

21. A method for treating obesity or reducing weight, **characterized by** comprising: inhibiting, with a Legl protein in an individual body as a target, a level of the Legl protein; or
blocking binding of the Legl protein to an epidermal growth factor receptor (EGFR) protein, with the Legl protein as a target; or
inhibiting activity of the Legl protein, with a Legl gene encoding the Legl protein as a target; or
inhibiting an expression level of the Legl gene, with a Legl gene encoding the Legl protein as a target; or
silencing an expression of the Legl gene by using an RNA interference vector capable of silencing expression of the Legl gene,
wherein an amino acid sequence of the Legl protein is as set forth in (1), (2) or (3):
(1) SEQ ID NO: 1;
(2) a derivative sequence that is obtained by subjecting a sequence as set forth in SEQ ID NO: 1 to substitution and/or deletion and/or addition of a plurality of amino acid residues and has a same or antagonizing bioactivity as SEQ ID NO: 1;
(3) a derivative sequence that is obtained by subjecting the sequence as set forth in SEQ ID NO: 1 to substitution, deletion or addition of one amino acid residue and has the same or antagonizing bioactivity as SEQ ID NO: 1.

22. A method for treating lipopenia or gaining weight, **characterized by** comprising: enhancing, with a Legl protein in an individual body as a target, a level of the Legl protein; or
promoting binding of the Legl protein to an epidermal growth factor receptor (EGFR) protein, with the Legl protein as a target; or
enhancing activity of the Legl protein, with the Legl protein as a target; or
enhancing an expression level of the Legl protein, with a Legl gene encoding the Legl protein as a target, wherein the amino acid sequence of the Legl protein is as set forth in (1), (2) or (3):
(1) SEQ ID NO: 1;
(2) a derivative sequence that is obtained by subjecting the sequence as set forth in SEQ ID NO: 1 to substitution and/or deletion and/or addition of a plurality of amino acid residues and has a same bioactivity as SEQ ID NO: 1;
(3) a derivative sequence that is obtained by subjecting the sequence as set forth in SEQ ID NO: 1 to substitution, deletion or addition of one amino acid residue and has a same bioactivity as SEQ ID NO: 1.

23. A method for treating human diabetes, **characterized by** comprising: enhancing, with a Legl protein in an individual body as a target, a level of the Legl protein; or
enhancing activity of the Legl protein to activate an Akt signal, so as to stimulate the transportation of GLUT2 to a surface of a cell membrane; or
activating the Akt signaling pathway by enhancing an expression level of a Legl gene encoding the Legl protein, so as to stimulate the transportation of GLUT2 to a surface of a cell membrane,
wherein an amino acid sequence of the Legl protein is as set forth in (1), (2) or (3):
(1) SEQ ID NO: 1;
(2) a derivative sequence that is obtained by subjecting a sequence as set forth in SEQ ID NO: 1 to substitution and/or deletion and/or addition of a plurality of amino acid residues and has a same bioactivity as SEQ ID NO: 1;
(3) a derivative sequence that is obtained by subjecting the sequence as set forth in SEQ ID NO: 1 to substitution, deletion or addition of one amino acid residue and has a same bioactivity as SEQ ID NO: 1.
